# EUROPEAN PATENT APPLICATION

(11) **EP 2 497 448 A1**
(43) Date of publication of application: **12.09.2012**
(21) Application number: 10840925.1
(22) Date of filing: 22.12.2010
(51) Int. Cl.: A61F 2/84

(54) **STENT DELIVERY SYSTEM**

(30) Priority: 28.12.2009 JP 2009298159
(71) Applicant: Terumo Kabushiki Kaisha, Tokyo 151-0072 (JP)
(72) Inventor: SUGIMOTO Ryota, Ashigarakami-gun Kanagawa 259-0151 (JP); KITAOKA Takashi, Ashigarakami-gun Kanagawa 259-0151 (JP)
(74) Representative: Prüfer & Partner GbR European Patent Attorneys
(86) International application number: PCT/JP2010/073149
(87) International publication number: WO 2011/081068

(57) **Abstract**

A stent delivery system (1) includes a self-expanding stent (10), an inner tube body (3), and a sheath (2) which contains the stent (10) within a distal portion thereof. The stent (10) can be discharged by moving the sheath (2) toward the proximal side relative to the inner tube body (3). The inner tube body (3) includes: a distal contact section (36) which is provided within a proximal portion of the stent (10) and does not enter side wall openings of the stent; and a proximal contact section (35) which is located at a position rear of the proximal end of the stent (10) and close to the distal contact section (36). The stent (10) has a proximal inwardly protruding section (17a) which can make contact with the distal contact section (36) of the inner tube body (3). The proximal inwardly protruding section is located between the distal contact section (36) and the proximal contact section (35). The stent (10) does not, except in the proximal portion thereof, have a bend free end which protrudes to the proximal side.

## Description

### Technical Field

The present invention relates to a stent delivery system for use in improvement of a stenosed part or occluded part generated in a living body lumen such as blood vessel, biliary duct, trachea, esophagus, and urethra.

### Background Art

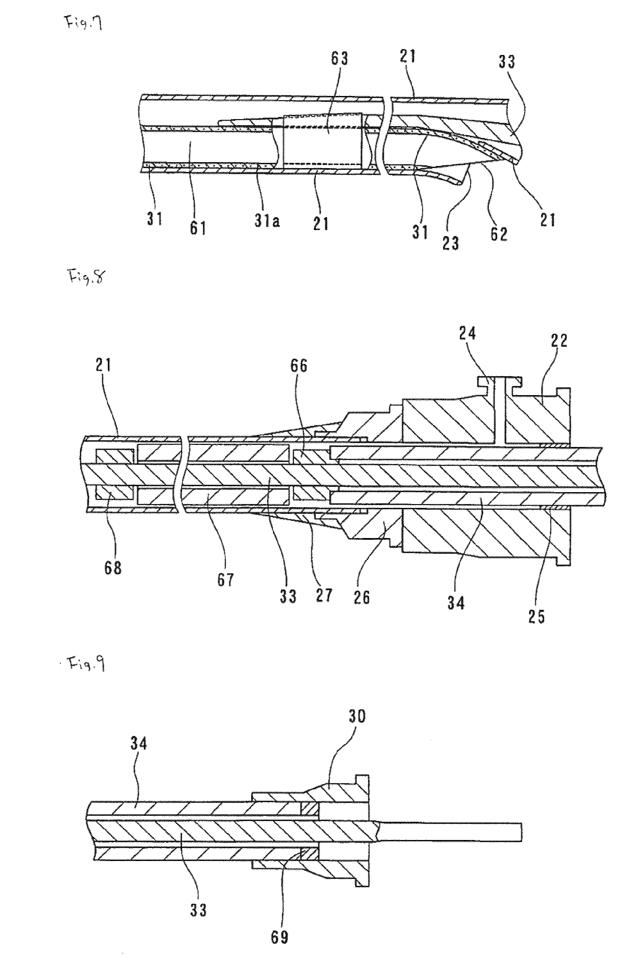

A stent delivery system has a stent for improvement of a stenosed part or occluded part. A stent is in general a tubular medical device which, for treating any of a variety of diseases generated through stenosis or occlusion of a blood vessel or other living body lumen, is put indwelling in the stenosed or obliterated part in order to dilate the stenosed or occluded part and secure the inner cavity there.
Now, description will be made below by taking a blood vessel as an example, which is not restrictive.
A stent is a body which, for insertion into a living body from the exterior, is small in diameter at the time of insertion, and is expanded in a target stenosed or occluded part to be enlarged in diameter and to maintain the lumen at that state.
In general, a stent is a hollow cylindrical member obtained by processing metallic wires or a metallic pipe. The stent is mounted to a catheter or the like in a radially reduced state, is inserted into a living body, and is expanded in a target part by some method to be put into secure contact with and fixed on the internal wall of the lumen at the target part, thereby maintaining the lumen shape. Stents are classified, according to the function and the method of placement, into self-expandable stents and balloon-expandable stents. A balloon-expandable stent is a stent which itself does not have an expanding function, and is used in such a manner that the stent mounted on a balloon is inserted into a target part, and thereafter the balloon is dilated to expand (elastically deform) the stent by the dilation force of the balloon, whereby the stent is put into secure contact with fixed on the internal surface of the lumen at the target part. This type of stent needs the work of expanding the stent as above-mentioned. On the other hand, a self-expandable stent is a stent which itself is provided with an expanding function, and is used in such a manner that the stent is inserted into a living body in a radially contracted state, and is relieved from contracted state in a target part, whereby the stent is allowed to expand by itself into the original expanded state so as to be put into secure contact with and fixed on the internal surface of the lumen, thereby maintaining the lumen shape.
The purpose of stent placement at present is for returning a blood vessel that has been stenosed for some reason into its original patent state. Thus, most of the stents are used mainly for preventing or restraining restenosis which might otherwise occur after such a surgical procedure as PTCA. In recent years, for further suppression of the probability of restenosis, drug-eluting stents with such a drug as immunosuppressant or carcinostatic agent loaded thereon have been used, and their effect has been known generally.
Most of the self-expandable stents are used in peripheral regions such as blood vessels in inferior limb or carotid artery, and there are self-expandable stents of the form as shown, for example, in Patent Document 1 (JP-T-H11-505441, WO96/26689).
Patent Document 2 (JP-T-2006-522654, WO2004/09145) discloses a stent delivery system including a catheter which has an inner shaft, at least a part of the inner shaft forming a stent-mounting region, a stent which has stent struts and is changed between a non-expanded state and an expanded state, the stent being disposed around at least a part of the stent-mounting region while in the non-expanded state, at least one band which forms at least a part of the stent-mounting region, the band being provided with a main body region having an external surface and a plurality of projections extending radially outward from the external surface, wherein when the stent-is in the non-expanded state, at least a part of the stent is disposed around and engaged with at least a part of the main body region of the band, and each of the projections has entered a space formed between the stent struts.
In addition, FIG. 8 discloses a stent delivery system in which a band 10 is provided at a distal portion of the stent and a proximal portion of the stent.

### Prior Art Documents

### Patent Documents

Patent Document 1: JP-T-H11-505441 (WO96/26689)
Patent Document 2: JP-T-2006-522654 (WO2004-091450)

### Summary of Invention

### Technical Problem

In the stent delivery system using the self-expandable stent as described in Patent Document 1, the self-expanding property possessed by the stent makes the positioning at the time of stent placement more difficult as compared with the case of a balloon-expandable stent. In addition, a jumping phenomenon in which the stent jumps out from the delivery system unguardedly may occur. If this phenomenon occurs, the stent would be disposed at a position deviated from a planned disposing position. Besides, in the procedure of placing the stent indwelling, readjustment of the indwelling position may be needed after the stent is discharged to some extent. In the stent delivery system described in Patent Document 1, however, it is difficult to re-contain the stent into the stent delivery system.
In the stent delivery system described in Patent Document 2, immediately upon exposure of a distal portion of the stent, the band on the distal side is spaced from the stent, so that only the band on the proximal side remains in engagement with the stent. When the sheath is pulled in this condition, all the frictional resistance between the stent and the sheath is loaded on the projections of the band on the proximal side, so that an excessive load is exerted also on the stent struts in engagement with the projections, which may result in breakage of the stent.
In addition, as a result of the present investors' earnest investigations, it was found out that where the projections have entered the spaces between the stent struts as in Patent Document 2, the sheath is caught on the projections to come into a locked state, which makes it difficult to pull the sheath. Besides, in the system described in Patent Document 2, it is necessary to match the spaces between the stent struts to the projections, so that the stent-containing work is difficult to carry out.
In consideration of the foregoing, it is an object of the present invention to provide a stent delivery system using a self-expandable stent, wherein a favorable stent-discharging operation is ensured, the stent can be again contained into the delivery system even after the stent is exposed from the delivery system to some extent, and it is easy to contain the stent into a stent-containing tube body.

### Technical Solution

The above-mentioned object can be attained by the following.
A stent delivery system including: a stent having a multiplicity of side wall openings, formed in a substantially hollow cylindrical shape, compressed toward a center axis thereof when inserted into a living body, and capable of being restored into a pre-compression shape by expanding outward when put indwelling in the living body; an inner tube body having a guide wire lumen; and a stent-containing tube body which contains the stent in a distal portion thereof, the stent being so disposed as to cover a distal portion of the inner tube body, and the stent being dischargeable by moving the stent-containing tube body toward a proximal side relative to the inner tube body,
wherein the inner tube body includes a distal contact section which is located in a proximal portion of the stent and which does not enter the side wall openings of the stent, and a proximal contact section which is disposed at a position rear of a proximal end of the stent and close to the distal contact section and which is capable of making contact with the proximal end of the stent; the stent is provided with a proximal inwardly protruding section capable of making contact with the distal contact section of the inner tube body; the stent is so disposed that the proximal inwardly protruding section is located between the distal contact section and the proximal contact section of the inner tube body; and the stent is provided with a distal portion and the proximal portion oriented respectively to a distal side and the proximal side of the stent-containing tube body and does not, except in the proximal portion thereof, have a bend free end which protrudes to the proximal side.

### Advantageous Effects

The stent delivery system according to the present invention is a stent delivery system including: a stent having a multiplicity of side wall openings, formed in a substantially hollow cylindrical shape, compressed toward a center axis thereof when inserted into a living body, and capable of being restored into a pre-compression shape by expanding outward when put indwelling in the living body; an inner tube body having a guide wire lumen; and a stent-containing tube body which contains the stent in a distal portion thereof, wherein the stent is so disposed as to cover a distal portion of the inner tube body, and the stent can be discharged by moving the stent-containing tube body toward the proximal side relative to the inner tube body.

Besides, in this stent delivery system, the inner tube body includes a distal contact section which is located within a proximal portion of the stent and which does not enter side wall openings of the stent, and a proximal contact section which is disposed at a position rear of the proximal end of the stent and close to the distal contact section and is capable of making contact with the proximal end of the stent; the stent has a proximal inwardly protruding section capable of making contact with the distal contact section of the inner tube body; and the stent is so disposed that the proximal inwardly protruding section is located between the distal contact section and the proximal contact section of the inner tube body. Further, in this stent delivery system, the stent includes a distal portion and the proximal portion oriented respectively to the distal side and the proximal side of the stent-containing tube body, and the stent does not, except in the proximal section thereof, have a bend free end protruding to the proximal side.

Therefore, a locked state of the stent which might arise from the distal contact section and the proximal contact section is prevented from occurring, and a favorable stent-discharging operation is ensured. Further, even after the stent is partly exposed from the stent-containing tube body, the stent can be again contained into the stent-containing tube body, owing to the engagement between the distal contact section of the inner tube body and the proximal inwardly protruding section of the stent, and the stent disposing position can be corrected. Accordingly, the stent can be assuredly disposed in a target part. In addition, the operation of containing the stent into the stent-containing tube body is easy to carry out.

### Brief Description of Drawings

[FIG. 1]

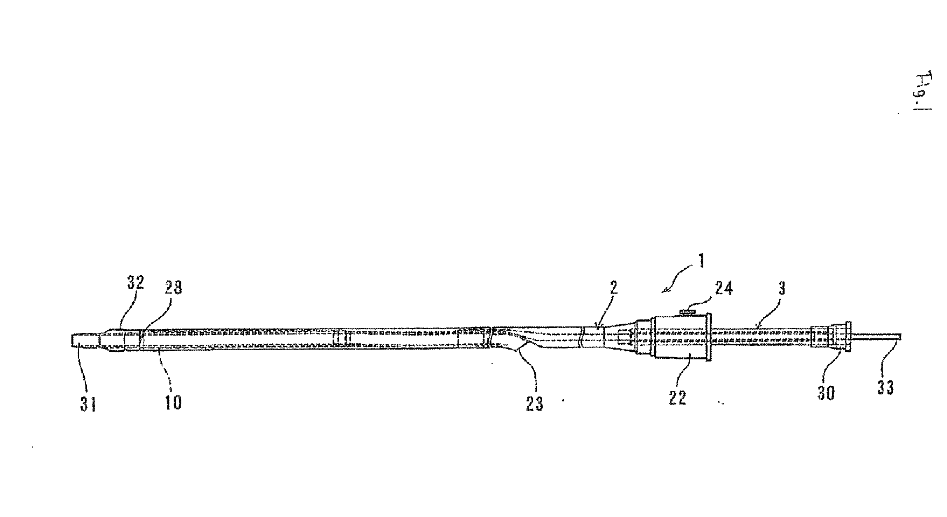

   Fig. 1 is a partly omitted front view of a stent delivery system according to an embodiment of the present invention.
[FIG. 2]

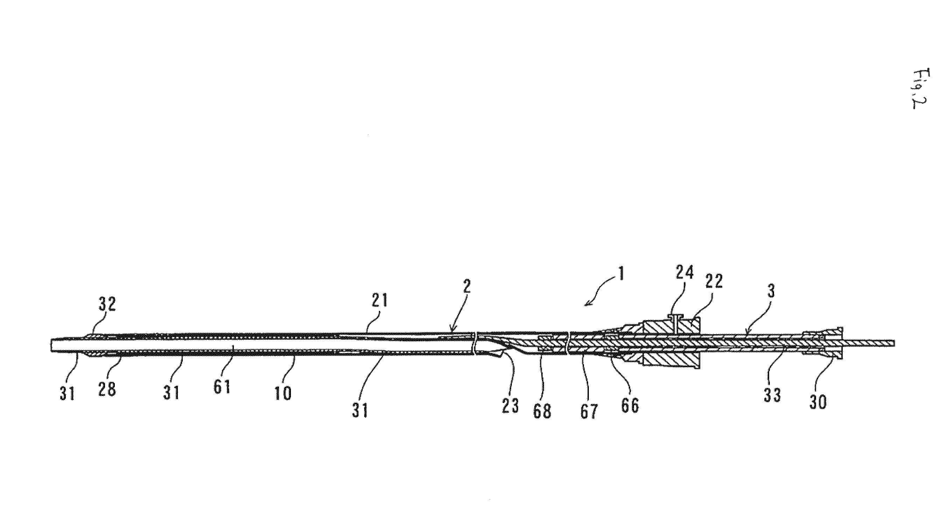

   FIG. 2 is a longitudinal sectional view of the stent delivery system shown in FIG. 1.
[FIG. 3]

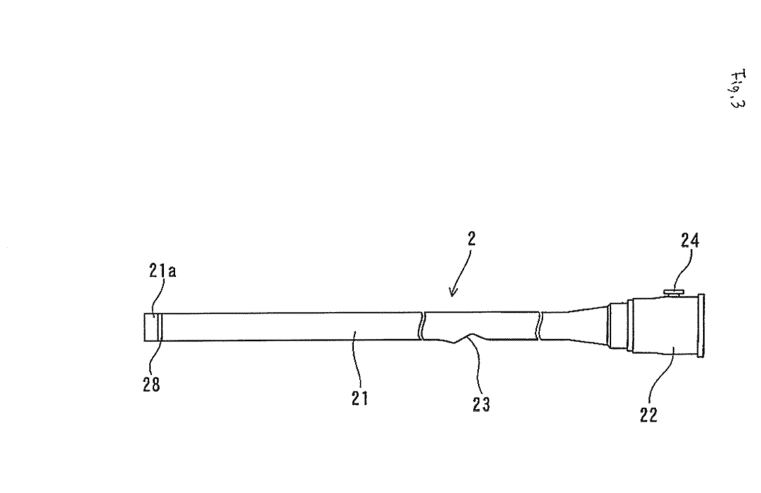

   FIG. 3 is a partly omitted front view of a stent-containing tube body (sheath) in the stent delivery system shown in FIG. 1.
[FIG. 4]

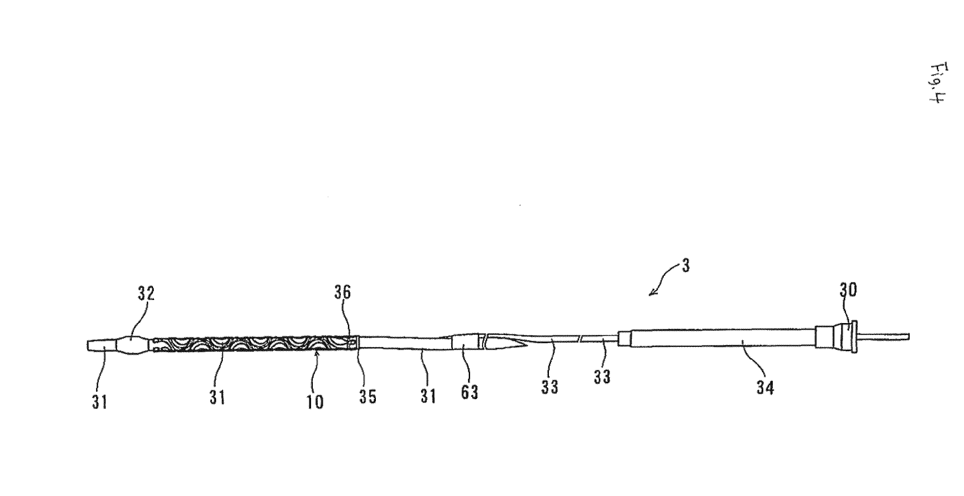

   FIG. 4 is a partly omitted front view of an inner tube body (inclusive of a stent) in the stent delivery system shown in FIG. 1.
[FIG. 5]

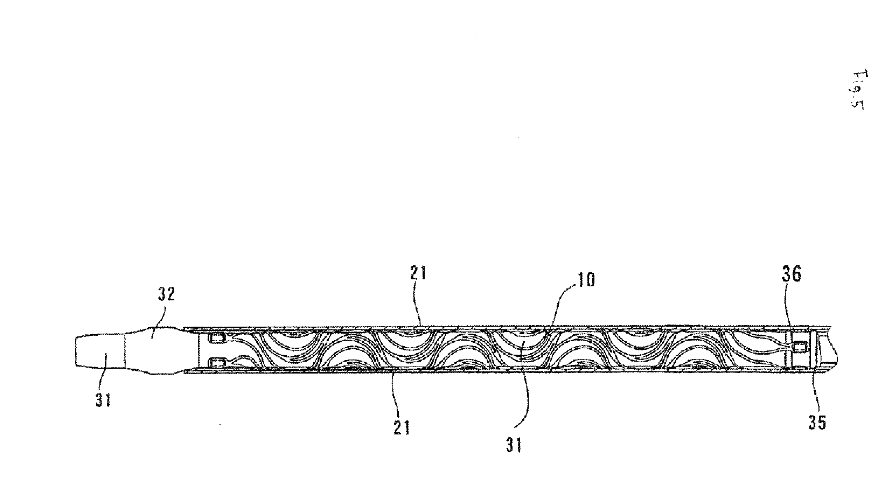

   FIG. 5 is an illustration of the vicinity of a distal portion of the stent delivery system shown in FIG. 1.
[FIG. 6]

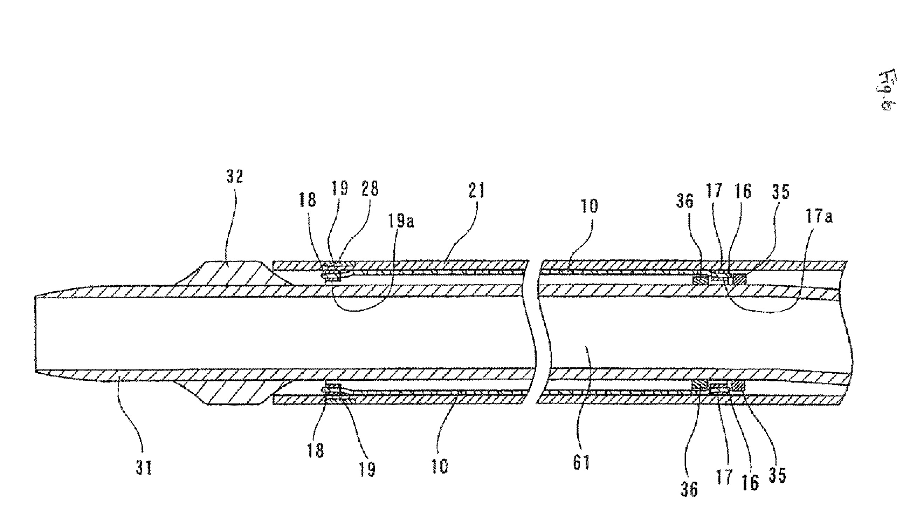

   FIG. 6 is a partly omitted enlarged sectional view of the distal portion of the stent delivery system shown in FIG. 1.
[FIG. 7]
   FIG. 7 is an illustration of an internal structure of the vicinity of an intermediate portion of the stent delivery system shown in FIG. 1.
[FIG. 8]
   FIG. 8 is a partly omitted enlarged sectional view of a proximal portion of the stent delivery system shown in FIG. 1.
[FIG. 9]
   FIG. 9 is a partly omitted enlarged sectional view of a proximal portion of the inner tube body in the stent delivery system shown in FIG. 1.
[FIG. 10]

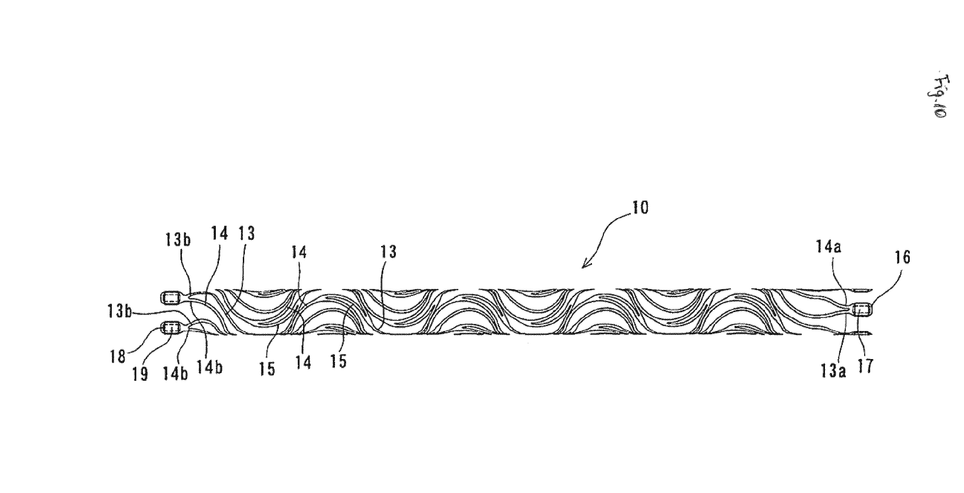

   FIG. 10 is a front view of an example of a stent for indwelling in a living body which is used in the stent delivery system according to the present invention.
[FIG. 11]

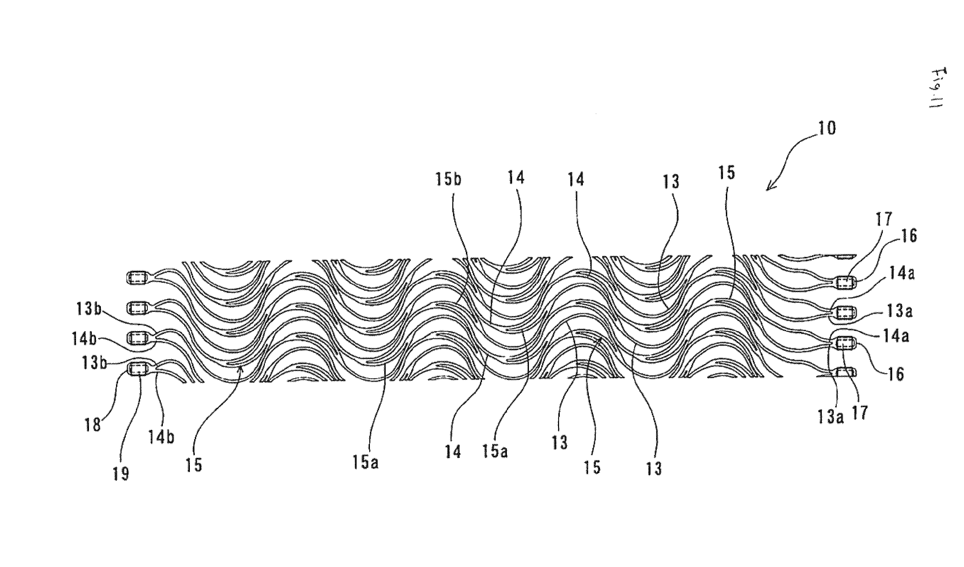

   FIG. 11 is a development of the stent for indwelling in a living body of FIG. 10.
[FIG. 12]

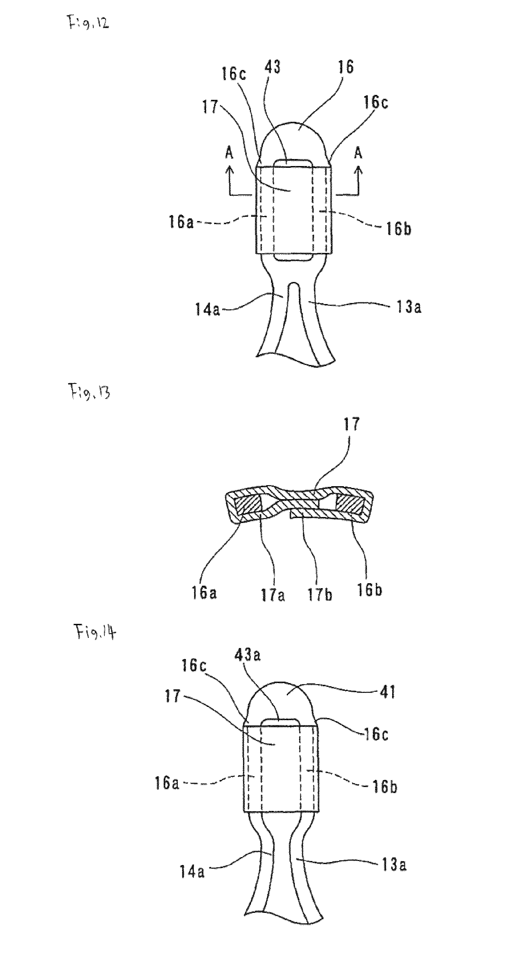

   FIG. 12 is an enlarged view of a proximal connection section of the stent for indwelling in a living body of FIG. 10.
[FIG. 13]
   FIG. 13 is a sectional view taken along line A-A of FIG. 12.
[FIG. 14]
   FIG. 14 is an enlarged view of a proximal connection section in another example of the stent for indwelling in a living body which is used in the stent delivery system according to the present invention.
[FIG. 15]

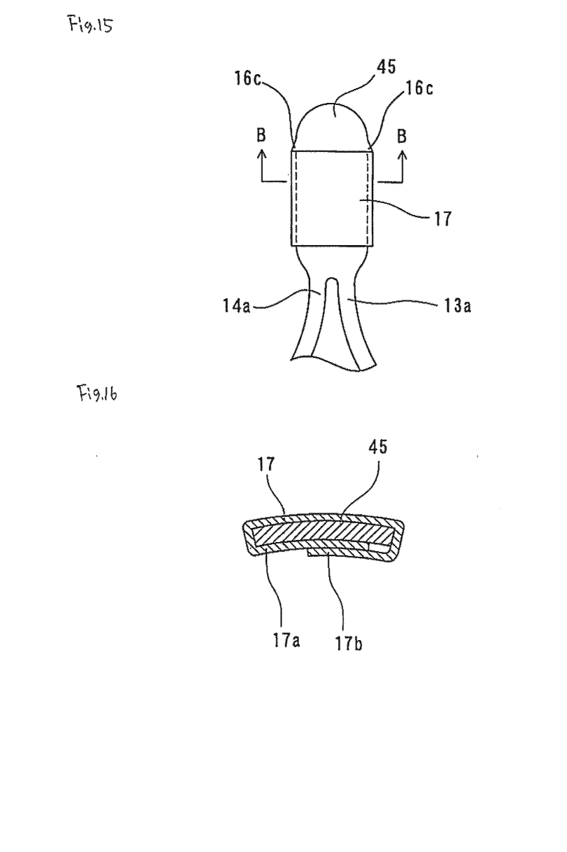

   FIG. 15 is an enlarged view of a proximal connection section in a further example of the stent for indwelling in a living body which is used in the stent delivery system according to the present invention.
[FIG. 16]
   FIG. 16 is a sectional view taken along line B-B of FIG. 15.
[FIG. 17]

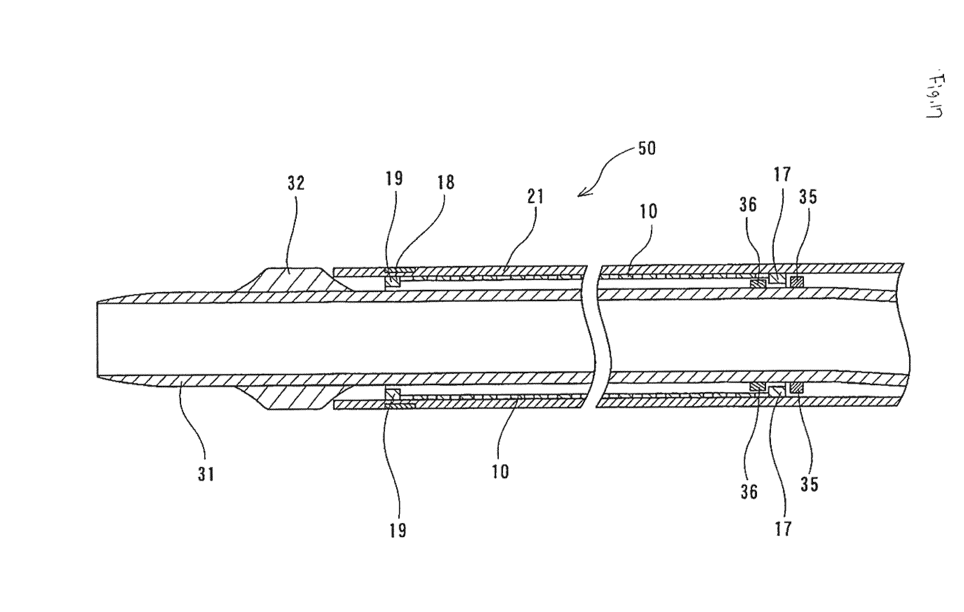

   FIG. 17 is a partly omitted enlarged sectional view of a distal portion of a stent delivery system according to another embodiment of the present invention.
[FIG. 18]

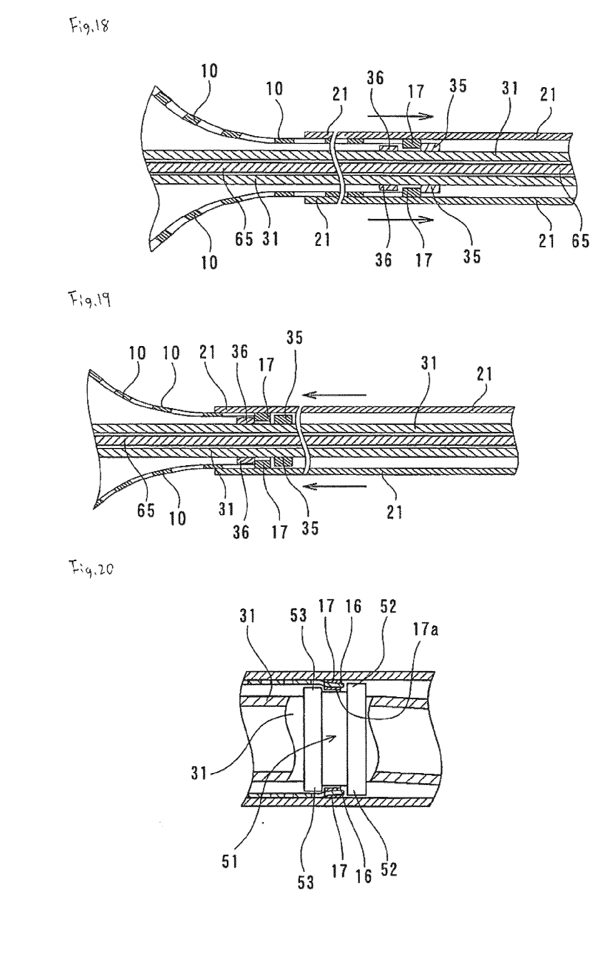

   FIG. 18 is an illustration of an operation of the stent delivery system according to present invention.
[FIG. 19]
   FIG. 19 is an illustration of an operation of the stent delivery system according to the present invention.
[FIG. 20]
   FIG. 20 is an illustration of a distal contact section and a proximal contact section in a stent delivery system according to a further embodiment of the present invention.
[FIG. 21]

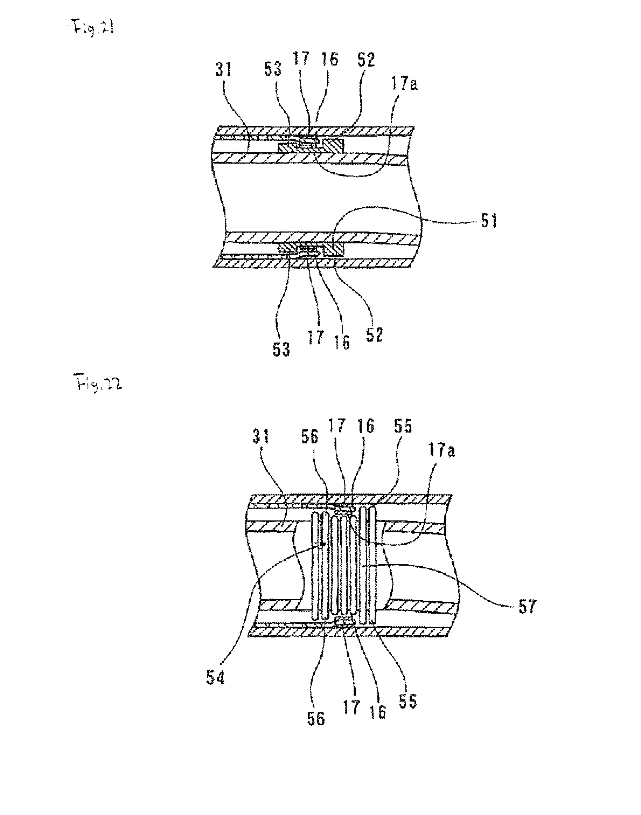

   FIG. 21 is a sectional view of FIG. 20.
[FIG. 22]
   FIG. 22 is an illustration of a distal contact section and a proximal contact section in a stent delivery system according to yet another embodiment of the present invention.
[FIG. 23]

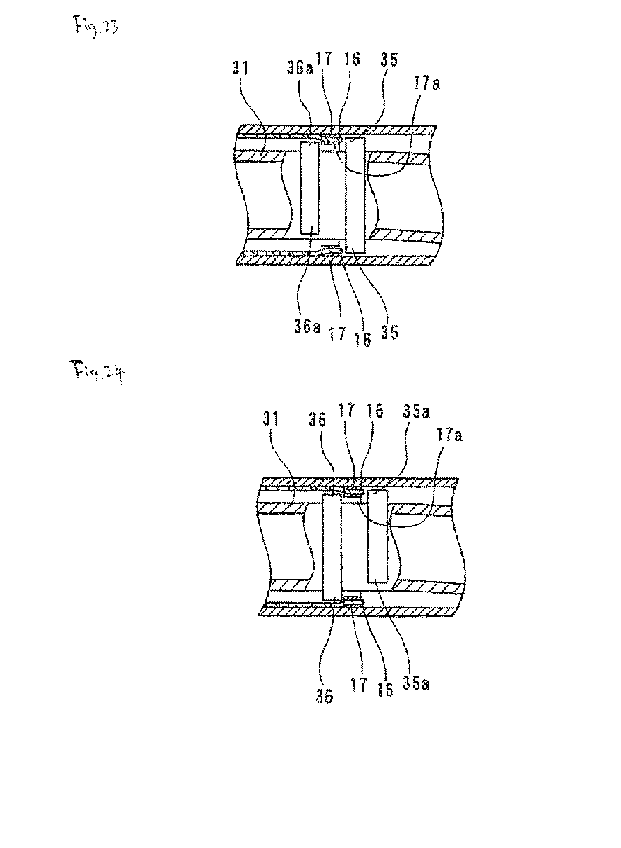

   FIG. 23 is an illustration of a distal contact section and a proximal contact section in a stent delivery system according to a yet further embodiment of the present invention.
[FIG. 24]
   FIG. 24 is an illustration of a distal contact section and a proximal contact section in a stent delivery system according to still another embodiment of the present invention.
[FIG. 25]

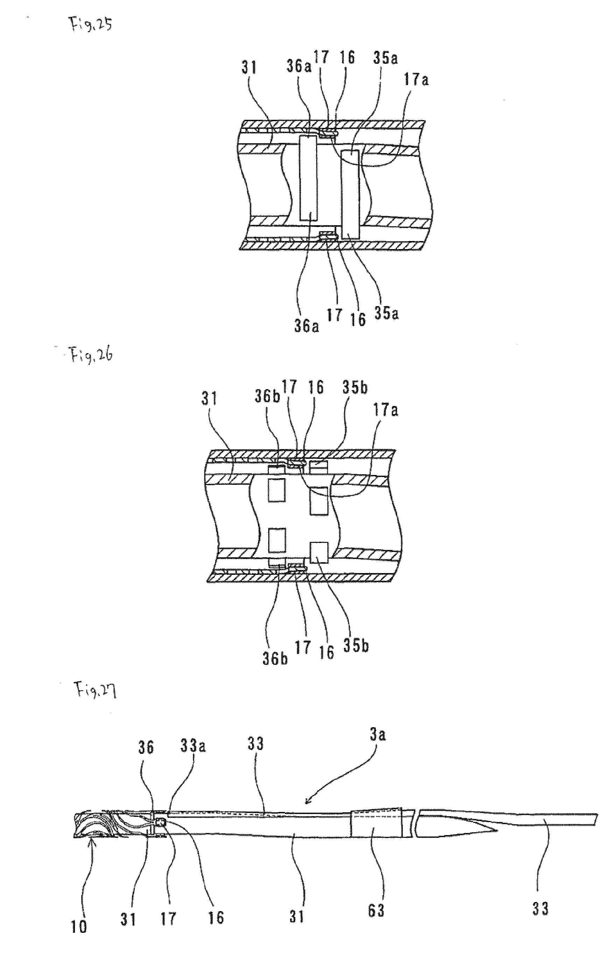

   FIG. 25 is an illustration of a distal contact section and a proximal contact section in a stent delivery system according to a still further embodiment of the present invention.
[FIG. 26]
   FIG. 26 is an illustration of a distal contact section and a proximal contact section in a stent delivery system according to another embodiment of the present invention.
[FIG. 27]
   FIG. 27 is an illustration of a central portion of an inner tube body (inclusive of a stent) in a stent delivery system according to a further embodiment of the present invention.
[FIG. 28]

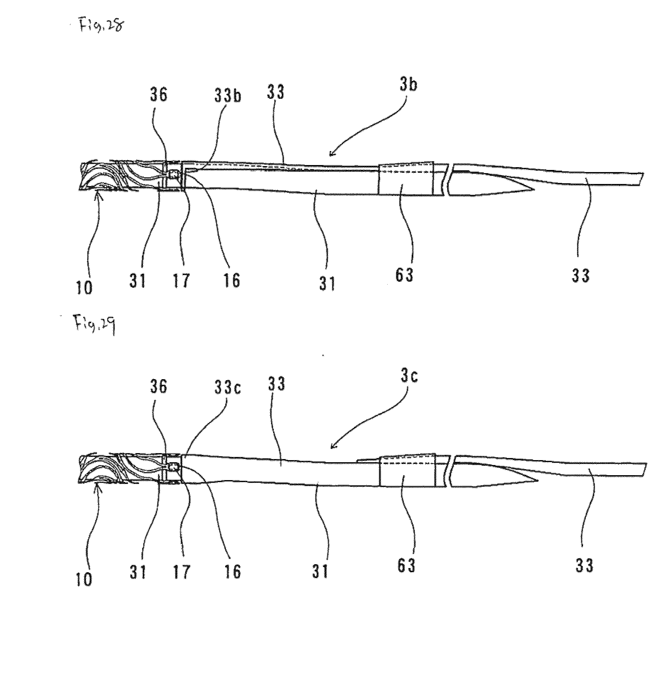

   FIG. 28 is an illustration of a central portion of an inner tube body (inclusive of a stent) in a stent delivery system according to yet another embodiment of the present invention.
[FIG. 29]
   FIG. 29 is an illustration of a central portion of an inner tube body (inclusive of a stent) in a stent delivery system according to a yet further embodiment of the present invention.
[FIG. 30]

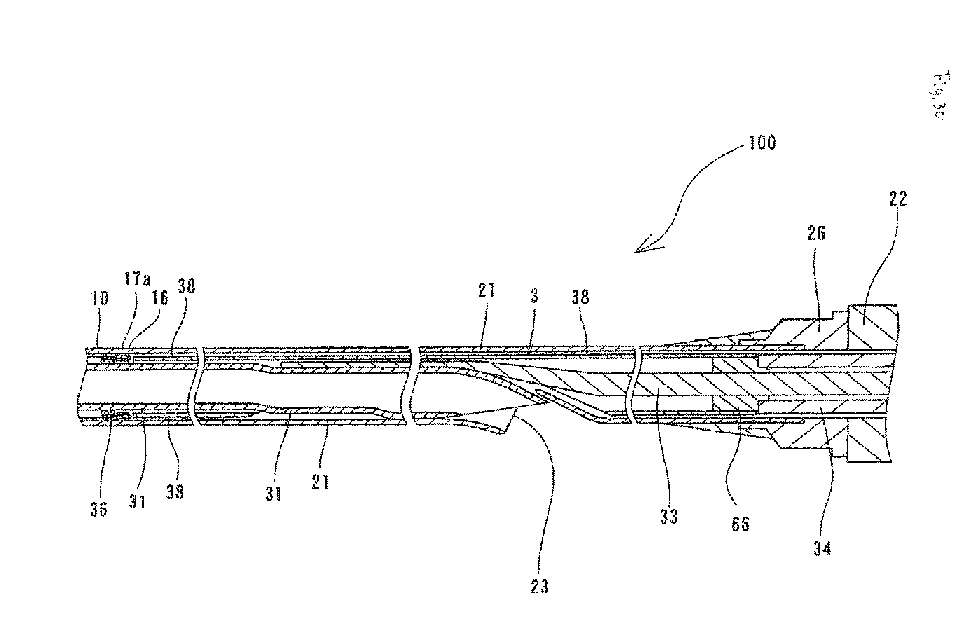

   FIG. 30 is an illustration of a stent delivery system according to still another embodiment of the present invention.
[FIG. 31]

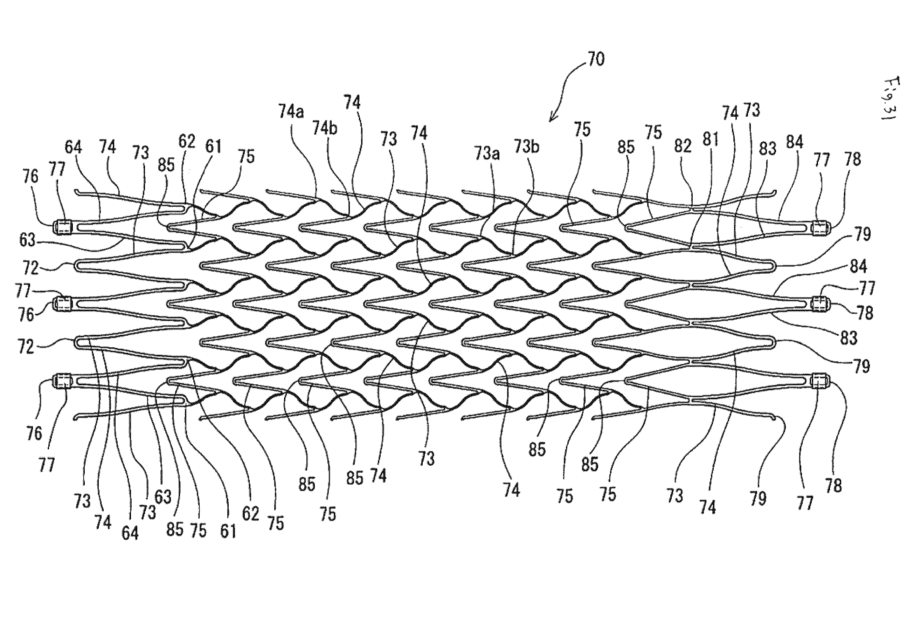

   FIG. 31 is a development of yet another example of the stent for indwelling in a living body which is used in the stent delivery system according to the present invention.
[FIG. 32]

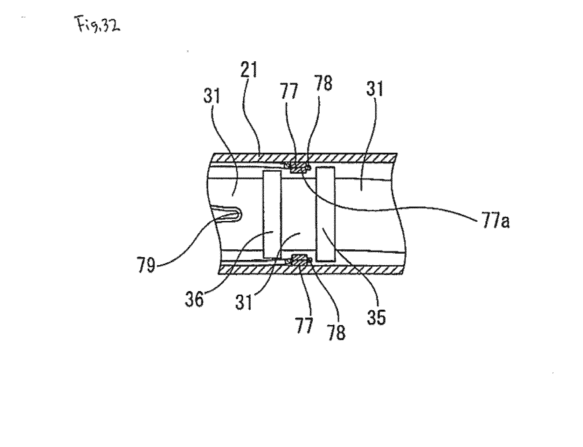

   FIG. 32 is an illustration of a stent delivery system using the stent for indwelling in a living body of FIG.

### Modes for Carrying Out the Invention

The stent delivery system according to the present invention will be described using the embodiments shown in the drawings.
The stent delivery system (in other words, living organ lesion improving instrument) 1 according to the present invention includes: a stent 10 having a multiplicity of side wall openings, formed in a substantially hollow cylindrical shape, compressed toward a center axis thereof when inserted into a living body, and capable of being restored into a pre-compression shape by expanding outward when put indwelling in the living body; an inner tube body (shaft section) 3 having a guide wire lumen 61; and a stent-containing tube body (sheath) 2 which contains the stent 10 in a distal portion thereof. In addition, the stent 10 is so disposed as to cover a distal portion of the inner tube body 3, and the stent 10 can be discharged by moving the stent-containing tube body 2 toward the proximal side relative to the inner tube body 3.
Besides, the inner tube body 3 includes: a distal contact section 36 which is located within a proximal portion of the stent 10 and which does not enter the side wall openings of the stent 10; and a proximal contact section 35 which is disposed at a position rear of a proximal end of the stent 10 and close to the distal contact section 36 and which is capable of making contact with the proximal end of the stent 10. In addition, the stent 10 has a proximal inwardly protruding section 17a capable of making contact with the distal contact section 36 of the inner tube body 3. Further, the stent 10 is so disposed that the proximal inwardly protruding section 17a is located between the distal contact section 36 and the proximal contact section 35 of the inner tube body 3.

In the stent delivery system 1 in the embodiment shown in the drawings, the inner tube body has the shaft section 3, and the stent-containing tube body has the sheath 2. Specifically, the stent delivery system 1 according to this embodiment includes: the stent 10 having the multiplicity of side wall openings, formed in a substantially hollow cylindrical shape, compressed toward the center axis thereof when inserted into a living body, and capable of being restored into a pre-compression shape by expanding outward when put indwelling in the living body; the shaft section 3 having the guide wire lumen 61; and the sheath 2 which contains the stent 10 in a distal portion thereof, wherein the stent 10 is so disposed as to envelope a distal portion of the shaft section 3.
In addition, the stent delivery system 1 according to the embodiment shown in the drawings includes: the stent 10 capable of being restored into a pre-compression shape by expanding outward when put indwelling in a living body; the stent-containing tube body (sheath) 2 which contains the stent 10 in a distal portion thereof; and the shaft section 3 which is slidably passed inside the stent-containing tube body (sheath) 2 and which is for discharging the stent 10 from the distal end of the stent-containing tube body (sheath) 2. The stent 10 has a distal portion and a proximal portion oriented respectively to the distal side and the proximal side of the stent-containing tube body (sheath) 2, does not, except in the proximal portion, substantially have a bend free end protruding to the proximal side, and is so configured that after a distal portion thereof is exposed from the stent-containing tube body (sheath) 2, the exposed portion can be again contained into the stent-containing tube body (sheath) 2 by moving the stent-containing tube body (sheath) 2 toward the distal side relative to the shaft section (inner tube body) 3. The stent delivery system 1 has the guide wire lumen 61 of which one end opens at the distal end of the stent delivery system and the other end opens on the proximal side relative to the stent-containing part of the sheath 2.

The stent delivery system 1 according to the present invention includes the stent 10, the sheath (stent-containing tube body) 2 which contains the stent 10 in a distal portion thereof, and the shaft section (inner tube body) 3 which is slidably passed inside the sheath 2.
As shown in FIGS. 1 to 8, the sheath (stent-containing tube body) 2 includes a sheath tube 21 and a sheath hub 22 fixed to the proximal end of the sheath tube 21.
As shown in FIGS. 1 to 8, the sheath tube 21 is a tubular body, and is opening at the distal end and the proximal end thereof. The distal opening functions as a port through which to discharge the stent 10, at the time of putting the stent 10 indwelling in a lesion part in a body cavity. By being discharged via the distal opening, the stent 10 is relieved from a stress load, and expands to be restored into its pre-compression shape. A distal portion of the sheath tube 21 forms the stent-containing part 21a which contains the stent 10 in the inside thereof. In addition, the sheath tube 21 has a side hole 23 provided on the proximal side relative to the stent-containing part 21a. The side hole 23 is for leading out a guide wire therethrough to the exterior.
In addition, a radiopaque marker 28 is preferably provided at the distal portion of the sheath (stent-containing tube body) 2. As shown in FIG. 6, the stent 10 which will be described later is contained in the tube body 3 so that the distal portion thereof is located substantially at the distal end of the radiopaque marker 28. The radiopaque marker 28 is preferably a tubular member made from a radiopaque material. As the material forming the radiopaque marker, there can be suitably used either one (simple substance) or at least two (alloy) selected from the group consisting of iridium, platinum, gold, rhenium, tungsten, palladium, rhodium, tantalum, silver, ruthenium, and hafnium.
The outside diameter of the sheath tube 21 is preferably 0.5 to 4.0 mm, particularly preferably 0.8 to 2.0 mm. The inside diameter of the sheath tube 21 is preferably, 0.2 to 1.8 mm, and the length of the sheath tube 21 is preferably 300 to 2500 mm, particularly preferably 300 to 2000 mm.
Taking the properties (flexibility, hardness, strength, slidability, anti-kinking properties, stretchability) required of the sheath tube into account, examples of the material which can be suitably used to form the sheath tube 21 include polyolefins such as polyethylene and polypropylene; nylon; polyethylene terephthalate; fluoro-polymers such as PTFE and ETFE; and thermoplastic elastomers. The thermoplastic elastomers are suitably selected from among nylon-based ones (e.g., polyamide elastomer), urethane-based ones (e.g., polyurethane elastomer), polyester-based ones (e.g., polyethylene terephthalate elastomer), and olefin-based ones (e.g., polyethylene elastomer, polypropylene elastomer).

Furthermore, the external surface of the sheath 2 is preferably subjected to a treatment for making the surface show lubricity. Examples of such a treatment include a method in which the external surface is coated with a hydrophilic polymer such as poly(2-hydroxyethyl methacrylate), polyhydroxyethyl acrylate, hydroxypropyl cellulose, methyl vinyl ether-maleic anhydride copolymer, polyethylene glycol, polyacrylamide, polyvinylpyrrolidone, or dimethylacrylamide-glycidyl methacrylate copolymer, and a method in which such a hydrophilic polymer is fixed to the external surface. Besides, the internal surface of the sheath tube 21 may be coated with such a hydrophilic polymer or the hydrophilic polymer may be fixed to the internal surface, to provide good slidability of the internal surface relative to the stent 10 and the shaft section 3.
In addition, the sheath hub 22 is fixed to a proximal portion of the sheath tube 21, as shown in FIGS. 1 to 3 FIG. 8. As shown in FIG. 8, the sheath hub 22 is provided with a seal member 25 which holds the shaft section 3 in a slidable and liquid-tight manner. Further, the sheath hub 22 is provided with a side port 24.
As the material constituting the sheath hub 22, a hard or semi-hard material is used. Examples of the hard or semi-hard material include synthetic resins such as polycarbonate, polyolefins (e.g., polyethylene, polypropylene, ethylene-propylene copolymer), styrene-based resins [e.g., polystyrene, MS resin (methacrylate-styrene copolymer), MBS resin (methacrylate-butylene-styrene copolymer)], and polyesters; and metals such as stainless steel, aluminum, and aluminum alloys.
Besides, as the materials constituting the seal member 25 and an elastic ring 69 to be described later, elastic materials are used. Examples of the elastic materials include rubbers such as synthetic rubbers, e.g., urethane rubber, silicone rubber, or butadiene rubber, and natural rubbers, e.g., latex rubber; and synthetic resin elastomers such as olefin-based elastomers (e.g., polyethylene elastomer, polypropylene elastomer), polyamide elastomers, styrene-based elastomers (e.g., styrene-butadiene-styrene copolymer, styrene-isoprene-styrene copolymer, styrene-ethylenebutylene-styrene copolymer), polyurethane, urethane-based elastomers, and fluoro-resin elastomers.
In addition, at the distal portion of the sheath hub 22 are provided reinforcement members 26 and 27 which extend toward the distal side beyond the distal end of the sheath hub.

As shown in FIGS. 1 to 6, the shaft section (inner tube body) 3 includes a shaft main body 33, a distal tube 31 which is provided at the distal end of the shaft main body 33 and which protrudes beyond the distal end of the sheath 2, and a shaft hub 30 fixed to a proximal portion of the shaft main body 33.
Besides, in this embodiment, the shaft section 3 is provided with a guide wire lumen proximal opening which opens at a side portion on the proximal side relative to the stent-containing part of the sheath 2, and the sheath 2 is provided with a sheath side hole on the proximal side relative to the stent-containing part, in such a manner that a guide wire can be inserted and passed via the sheath side hole and the proximal opening.
As shown in FIG. 5, the distal tube 31 protrudes beyond the distal end of the sheath 2. In addition, the distal tube 31 is provided with a stopper 32 which inhibits the sheath 2 from moving in the distal direction. As shown in FIG. 7, a proximal portion of the distal tube 31 is bent, enters the side hole 23 of the sheath tube 21, and is disengageably engaged with the side hole 23. The outside diameter of the distal tube 31 is preferably 0.2 to 1.8 mm. Further, a distal portion of the distal stopper 32 is preferably decreased in diameter toward the distal side, as shown in FIG. 5. The outside diameter of the maximum diameter portion of the stopper 32 is preferably 0.5 to 4.0 mm. In addition, a proximal portion of the stopper 32 is also preferably decreased in diameter toward the proximal side, as shown in FIG. 5. Besides, the distal tube 31 has the guide wire lumen 61 extending from the distal end to the proximal end thereof, and the position of the proximal opening 62 is preferably located 10 to 400 mm, more preferably 50 to 350 mm, to the proximal side relative to the distal end of the distal tube 31. In addition, the position of the proximal opening 62 is preferably about 50 to 250 mm to the proximal side relative to the proximal end of the stent 10 disposed (in other words, relative to the proximal end of the stent-containing part).

The stent 10 to be used in the present invention is a so-called self-expandable stent which has a multiplicity of openings in the side surface thereof and which can be restored into a pre-compression shape by expanding outward when put indwelling in a living body. Further, the stent 10 has a configuration in which the stent 10 has a distal portion and a proximal portion oriented respectively to the distal side and the proximal side of the sheath 2. The stent 10 does not, except in the proximal portion, have a bend free end protruding to the proximal side (in other words, being oriented toward the proximal side), and, after a distal portion of the stent 10 is exposed from the sheath 2, the exposed distal portion can be again contained into the sheath 2 by moving the sheath 2 toward the distal side relative to the shaft section 3.
The stent to be used may be a stent which does not have a free end, owing to a structure in which a vertex of a proximal bend section or a portion near the vertex is connected to other linear component element. Besides, the stent to be used may be as shown in FIGS. 10 and 11. FIG. 10 is a front view of an example of a stent for indwelling in a living body which is used in the stent delivery system according to the present invention. FIG. 11 is a development of the stent for indwelling in a living body of FIG. 10.
The stent 10 includes wavy struts 13, 14 extending in the axial direction from one end to the other end of the stent and being arrayed in plurality along the circumferential direction of the stent, and one or a plurality of connection struts 15 interconnecting each pair of adjacent wavy struts and extending over a predetermined length in the axial direction. The end portions of the wavy struts 13, 14 are each connected to an end portion of an adjacent wavy strut. In addition, the stent 10 is provided with a multiplicity of openings formed between the struts.

Particularly, the stent 10 shown in FIGS. 10 and 11 includes first wavy struts 13 extending in the axial direction from one end to the other end of the stent 10 and being arrayed in plurality in the circumferential direction of the stent, second wavy struts 14 being each located between the first wavy struts 13, extending in the axial direction from one end to the other end of the stent and being arrayed in plurality in the circumferential direction of the stent, and one or a plurality of connection struts 15 interconnecting each pair of the first wavy strut 13 and the second wavy strut 14 adjacent to each other and extending over a predetermined length in the axial direction. Besides, the vertex of the second wavy strut 14 is deviated by a predetermined length in the axial direction of the stent, relative to the vertex of the first wavy strut 13 adjacent to the second wavy strut 14 in the circumferential direction of the stent 10 and bent to the same direction as the second wavy strut 14. In addition, end portions 13a and 13b of the first wavy strut 13 are connected respectively to end portions 14a and 14b of the second wavy strut adjacent to the first wavy strut 13.
The stent 10 of this example is a so-called self-expandable stent having a substantially hollow cylindrical shape, compressed toward its center axis when inserted into a living body and being restored into its pre-compression shape by expanding outward when put indwelling in the living body.

The first wavy struts 13 extend in an axial direction substantially in parallel to the center axis of the stent. Besides, the first wavy struts 13 are arrayed in plurality in the circumferential direction of the stent. The number of the first wavy struts 13 is preferably not less than three, and particularly preferably three to eight. Furthermore, the plurality of the first wavy struts 13 are preferably disposed at substantially regular angular intervals around the center axis of the stent.
The second wavy struts 14 also extend in an axial direction substantially in parallel to the center axis of the stent. Besides, the second wavy struts 14 are arrayed in plurality in the circumferential direction of the stent, and the second wavy struts 14 are each disposed between the first wavy struts. The number of the second wavy struts 14 is preferably not less than three, and particularly preferably three to eight. Furthermore, the plurality of the second wavy struts are preferably disposed at substantially regular angular intervals around the center axis of the stent. In addition, the number of the second wavy struts 14 is the same as the number of the first wavy struts 13.
Besides, this stent 10 has one or a plurality of connection struts 15 interconnecting each pair of the first wavy strut 13 and the second wavy strut 14 adjacent to each other and extending over a predetermined length in the axial direction. Especially, in the stent 10 in this example, the connection strut 15 has its one end in the vicinity of an inflection point of the wavy strut on one side, has its other end in a region ranging from the vicinity of a vertex to a point located a little beyond the vertex of the adjacent wavy strut on the other side, extends in the axial direction, and is bent to the same direction as the vertex of the wavy strut on the other side. Specifically, as shown in FIG. 11, the connection strut 15 includes a bent first connection strut 15a having a vertex directed toward one side in the circumferential direction of the stent 10, and a bent second connection strut 15b having a vertex directed toward the other side in the circumferential direction of the stent 10. In addition, the connection strut 15 is bent in an arcuate shape, and has a radius approximately equal to the radius of the arc of a bent portion of the first wavy strut 13 or second wavy strut 14 which is adjacent thereto in the circumferential direction of the stent 10.

The stent 10 in this example has coupling sections 16, 18 which respectively couple the end portion on one side and that on the other side of every first wavy strut with an end portion of either one of the second wavy struts adjacent thereto. Specifically, an end portion 13a on the one side of the first wavy strut of the stent 10 is coupled by the coupling section 16 to an end portion 14a on the one side of the adjacent second wavy struts 14 on one side (specifically, the second wavy strut 14 adjacent to the first wavy strut and located on the other side in the circumferential direction). Meanwhile, an end portion 13b on the other side of the first wavy strut is coupled by the coupling section 18 to an end portion 14b on the other side of the adjacent second wavy struts 14 on the other side (specifically, the second wavy strut 14 adjacent to the first wavy strut and located on the one side in the circumferential direction). In other words, the coupling section 16 on the one end and the coupling section 18 on the other end each couple a different pair of a first wavy strut 13 and a second wavy strut 14 (the combination shifts by one line).

In addition, as shown in FIG. 6, the stent 10 has the proximal inwardly protruding section 17a which can make contact with the distal contact section 36 of the inner tube body 3 to be described later. The proximal inwardly protruding section 17a of the stent 10 is so disposed as to be located between the distal contact section 36 and the proximal contact section 35 of the inner tube body 3 to be described later. The proximal inwardly protruding section 17a is preferably formed of the radiopaque marker (a marker opaque to radiations) 17 mounted to the proximal portion (the coupling section) 16 of the stent 10. Besides, as shown in FIG. 6, the proximal inwardly protruding section 17a of the stent 10 does not make contact with the external surface of the distal tube 31 of the shaft section (inner tube body) 3. Incidentally, as in a stent delivery system 50 in an embodiment shown in FIG. 17, the proximal inwardly protruding section of the stent 10 may be a thick-walled section formed of the proximal portion (the coupling section) 16 of the stent. The protrusion height of the proximal inwardly protruding section is preferably 0.05 to 0.2 mm. In addition, the difference in height between the proximal inwardly protruding section and the other non-protruding section of the stent 10 is preferably 0.01 to 0.1 mm.
Furthermore, as shown in FIG. 6, the stent 10 in this example may have a distal inwardly protruding section 19a at the distal portion thereof. The distal inwardly protruding section 19a preferably is formed of a radiopaque marker 19 mounted to the distal portion (the coupling section) 18 of the stent 10. Incidentally, as in the stent delivery system 50 in the embodiment shown in FIG. 17, the distal inwardly protruding section of the stent 10 may be a thick-walled section formed of the distal portion (the coupling section) 18 of the stent.

In the stent in this example, the radiopaque markers 17 are attached to the coupling sections 16. In this example, the coupling section 16 has an opening 43, and has two frame portions 16a and 16b extending in parallel in the direction toward the proximal end of the stent (toward the end portion of the coupling section) with a predetermined space therebetween. The radiopaque marker 17 covers substantially the whole part of the two frame portions 16a and 16b. In addition, the proximal inwardly protruding section 17a of the stent 10 is constituted by that portion of the radiopaque marker 17 which is on the inner side of the stent. Further, in the stent in this example, as shown in FIGS. 12 and 13, the proximal inwardly protruding section 17a of the stent 10 is formed of that portion of a sheet-shaped member wound around the opening 43 of the proximal portion (coupling section) of the stent 10 which is on the inner side of the stent. Furthermore, in the stent in this example, the sheet-shaped member has an inner overlap section 17b which protrudes to the inner side of the stent 10, thereby forming a section protruding more than the other section. The radiopaque marker 17 forming the proximal inwardly protruding section preferably has a predetermined thickness (wire diameter). Besides, in the structure shown in FIGS. 12 and 13, the radiopaque marker 17 contains therein the two frame portions forming the proximal portion (coupling section) 16, is depressed in a central portion thereof, and is partly overlapped on itself, to be thereby fixed to the two frame portions. Incidentally, the proximal portion (coupling section) of the stent may, as shown in FIG. 14, not have an independent opening. In the structure shown in this example, the proximal end of the end portion 14a of the strut is continuous with the frame portion 16a of the proximal portion 41, and the proximal end of the end portion 13a of the strut is continuous with the frame portion 16b of the proximal portion 41. Therefore, an opening 43a opens at one end portion thereof and communicates with the space between the two struts. Furthermore, the proximal portion (coupling section) of the stent may, as shown in FIGS. 15 and 16, not have the above-mentioned opening 43. In the stent in this example, a coupling section 45 is a slightly curved plate-shaped section wish a predetermined area, and the radiopaque marker 17 is so mounted as to cover the face and back sides of the coupling section 45. Further, in the stent in this example, as shown in FIGS. 15 and 16, the proximal inwardly protruding section 17a of the stent 10 is formed of that portion of the sheet-shaped member wound around the proximal portion (coupling section) 45 of the stent 10 which is on the inner side of the stent. Furthermore, in the stent in this example, the sheet-shaped member has an inner overlap section 17b which protrudes to the inner side of the stent 10, thereby forming a section protruding more than the other section.

As the scents in the examples shown in FIGS. 12 to 16, the stent's proximal portion is preferably provided with a stopper section or sections 16c by which the radiopaque marker 17 forming the proximal inwardly protruding section of the stent 10 is inhibited from moving in the proximal direction. Particularly, as shown in FIGS. 12, 14 and 15, two stopper sections 16c are provided on opposite sides. Where such stopper sections are provided, it is ensured that when the radiopaque marker 17 is pressed toward the stent's proximal side by the distal contact section 36 of the inner tube body 3 (described later) at the time of re-containing the stent 10 into the sheath 2, the radiopaque marker 17 can be prevented from being moved relative to the stent or disengaged from the stent. Besides, in the stents in the examples shown in FIGS. 12 to 16, the stent's proximal portion 16, 41, 45 protrudes to the proximal side relative to the radiopaque marker 17. This ensures that at the time of discharging the stent, the proximal contact section 35 of the inner tube body 3 makes contact with the proximal end of the proximal portion 16 of the stent 10, and the proximal contact section 35 of the inner tube body 3 does not make contact with the radiopaque marker 17, so that the radiopaque marker 17 is prevented from being moved relative to the stent or disengaged from the stent.
Incidentally, in all the examples mentioned above, it is preferable to use the above-mentioned sheet-shaped member as the radiopaque marker, however, one formed by winding a wire-shaped member around the proximal portion (coupling section) of the stent may also be used. In this case as well, the radiopaque marker is preferably provided with an inner overlap section which protrudes to the inner side of the stent. As the material forming the above-mentioned radiopaque marker, use can be suitably made of one (simple substance) or at least two (alloy) selected from the group consisting of iridium, platinum, gold, rhenium, tungsten, palladium, rhodium, tantalum, silver, ruthenium, and hafnium.
Fixation of the radiopaque marker can be carried out by means of any of welding, soldering, adhesion, fusing, and diffusion.

The material constituting the stent 10 is preferably a superelastic metal. As the superelastic metal, superelastic alloys are preferably used. The superelastic alloys here mean those alloys which are generally called shape memory alloys and which show superelasticity at least at a living body temperature (around 37°C). Particularly, such superelastic alloys as Ti-Ni alloys containing 49 to 53 atomic% of Ni, Cu-Zn alloys containing 38.5 to 41.5 wt.% of Zn, Cu-Zn-X alloys (X = Be, Si, Sn, Al, Ga) containing 1 to 10 wt.% of X, and Ni-Al alloys containing 36 to 38 atomic% of Al are preferably used. Especially preferred the above Ti-Ni alloys. Besides, mechanical properties of the superelastic alloys can be changed, as required, by replacing part of the Ti-Ni alloys with 0.01 to 10.0% of X to obtain Ti-Ni-X alloys (X = Co, Fe, Mn, Cr, V, Al, Nb, W, B or the like), by replacing part of the Ti-Ni alloys with 0.01 to 30.0% of X to obtain Ti-Ni-X alloys (X = Cu, Pb, Zr), or by selecting a cold working rate and/or final heat treatment conditions. Further, by selecting the cold working rate and/or final heat treatment conditions while using the Ti-Ni-X alloys, it is possible to change the mechanical properties of the alloys as required. The buckling strength (yield stress when loaded) of the superelastic alloy used is preferably 5 to 200 kg/mm² (22°C), more preferably 8 to 150 kg/mm², and the restoring stress (yield strength when unloaded) of the superelastic alloys is preferably 3 to 180 kg/mm² (22°C), more preferably 5 to 130 kg/mm². The superelasticity here means a property of a material such that even when the material is deformed (bent, stretched or compressed) into a region in which ordinary metals are plastically deformed at a use temperature, the deformed material is restored substantially into the pre-compression shape after the deformation is released, without need for heating.
The diameter of the stent when compressed is preferably 0.5 to 1.8 mm, particularly preferably 0.6 to 1.4 mm. The length of the stent when non-compressed is preferably 5 to 200 mm, particularly preferably 8.0 to 100.0 mm. The diameter of the stent when non-compressed is preferably 1.5 to 6.0 mm, particularly preferably 2.0 to 5.0 mm. Further, the wall thickness of the stent is preferably 0.05 to 0.15 mm, particularly preferably 0.05 to 0.40 mm. The width of the wavy struts is preferably 0.01 to 1.00 mm, particularly preferably 0.05 to 0.2 mm. The surfaces of the wavy struts are preferably smooth surfaces obtained by processing, more preferably surfaces smoothened by electrolytic polishing. In addition, the radial-directional strength of the stent is preferably 0.1 to 30.0 N/cm, particularly preferably 0.5 to 5.0 N/cm.

As shown in FIGS. 4, 5 and 6 (particularly, FIG. 6), the inner tube body 3 includes: the distal contact section 36 which is located within the proximal portion of the stent 10 and does not make contact with the internal surface of the stent-containing tube body 2; and the proximal contact section 35 which is disposed at a position rear of the proximal end of the stent 10 and close to the distal contact section 36, does not make contact with the internal surface of the stent-containing tube body 2 and can make contact with the proximal end of the stent 10. Besides, in this embodiment, as shown in FIGS. 4, 5 and 6 (particularly, FIG. 6), the distal contact section 36 is a distal protruding section which protrudes from the external surface of the distal tube 31, and the proximal contact section 35 is a proximal protruding section which also protrudes from the external surface of the distal tube 31 as with the distal contact section 36.
The proximal inwardly protruding section 17a of the stent 10 as above-mentioned is capable of making contact with the distal contact section 36 of the inner tube body 3. In addition, as shown in FIG. 6, the inwardly protruding section 17a of the stent 10 is located between the distal contact section 36 and the proximal contact section 35 of the inner tube body 3.
As shown in FIGS. 5 and 6, the stent delivery system 1 in this embodiment has the distal contact section 36 at a position spaced a predetermined distance to the proximal side relative to the distal end of the distal tube 31. The distal contact section 36 is disposed at a position which is within the proximal portion of the stent 10 and which is slightly on the distal side relative to the proximal end of the stent 10. In addition, the proximal contact section 35 is provided at a position slightly on the proximal side relative to the distal contact sections 36. The proximal contact section 35 is disposed in the vicinity of and on the rear side (proximal side) of the proximal end of the stent 10. The proximal inwardly protruding section 17a of the stent 10 is located between the distal contact section 36 and the proximal contact section 35 of the inner tube body 3. Therefore, the distance between the distal contact section 36 and the proximal contact section 35 is a little longer than the axial length of the proximal inwardly protruding section 17a of the stent 10. The distance between the distal contact section 36 and the proximal contact section 35 is longer than the axial length of the proximal inwardly protruding section 17a of the stent 10, preferably by 0.02 to 1.0 mm, particularly preferably by 0.05 to 0.3 mm.

The distal contact section 36 does not enter the side wall openings of the stent 10. Furthermore, the distal contact section 36, preferably, does not substantially make contact with the internal surface of the stent 10. In addition, the distal contact section 36 is preferably an annular protruding section provided continuously over the outer circumference of the distal tube. 31. The annular protruding section is formed, for example, by attaching a tubular member to the outer circumference of the distal tube. With the distal contact section 36 set to be such an annular protruding section, its contact with the proximal inwardly protruding section 17a of the stent 10 becomes assured. Besides, the distal contact section 36, preferably, does not substantially make contact with the internal surface of the stent 10. This ensures that the distal contact section 36 would not become an obstacle at the time of discharging the stent. The distal contact section 36 has such a height as to be able to make contact with the proximal inwardly protruding section 17a of the stent 10. The height of the distal contact section 36 is preferably 0.06 to 0.11 mm, particularly preferably 0.08 to 1.1 mm. In addition, the axial length of the distal contact section 36 is preferably 0.1 to 3.0 mm, particularly preferably 0.3 to 2.0 mm.
Incidentally, while the distal contact section 36 is preferably an annular protruding section provided continuously over the outer circumference of the distal tube 31, it may be a plurality of non-continuous ribs which are arranged in an annular pattern.
Besides, the proximal contact section 35 is preferably an annular protruding section provided continuously over outer circumference of the distal tube 31. The annular protruding section is formed, for example, by attaching a tubular member to the outer circumference of the distal tube. In addition, the proximal contact section 35, preferably, does not make contact with the internal surface of the sheath 2. This ensures that the proximal contact section 35 would not become an obstacle at the time of discharging the stent. The proximal contact section 35 has such a height as to be able to make contact with the proximal end of the stent 10. The height of the proximal contact section 35 is preferably 0.08 to 0.18 mm, particularly preferably 0.1 to 0.16 mm. The axial length of the proximal contact section 35 is preferably 0.1 to 3.0 mm, particularly preferably 0.3 to 2.0 mm. The distance between the proximal contact section 35 and the internal surface of the sheath is preferably 0.01 to 0.04 mm. Besides, preferably, the proximal contact section 35 is higher than the distal contact section 36, with the difference in height between them being 0.02 to 0.1 mm.
Furthermore, the distal contact section 36 and the proximal contact section 35 are preferably made from a radiopaque material. As the radiopaque material, the materials for forming the radiopaque marker as above-mentioned can be used suitably. Particularly, these contact sections are each preferably formed by attaching a radiopaque tubular member. Furthermore, the distal contact section 36 and the proximal contact section 35, preferably, are each made from a radiopaque material and are different from each other in axial length. This enables easy discrimination between the two contact sections. While either of the two contact sections may be longer, the difference in length between them is preferably 0.3 to 1.0 mm.

In the stent delivery system 1 in this embodiment, the inner tube body 3 (specifically, the distal tube 31) has an opening 62 through which a guide wire is led out to the exterior on the proximal side relative to the stent-containing part of the stent-containing tube body 2.
In addition, the distal tube 31 is preferably provided with a reinforcement layer 31a at least at a part located on the proximal side relative to the proximal portion of the stent. In the structure of this embodiment, the reinforcement layer 31a is provided over the whole part of the distal tube 31. Incidentally, the reinforcement layer 31a may not be provided at a distalmost part of the distal tube 31. The reinforcement layer 31a is preferable a net-formed reinforcement layer. The net-formed reinforcement layer is preferably formed by use of braid wires. The braid wires are, for example, wire braid, which can be formed from metallic wires of stainless steel, an elastic metal, a superelastic alloy, a shape memory alloy or the like having a wire diameter of 0.01 to 0.2 mm, preferably 0.03 to 0.1 mm. Besides, the net-formed reinforcement layer may be formed from synthetic fibers such as polyamide fibers, polyester fibers, and polypropylene fibers.

The shaft main body 33 includes a distal portion which is fixed to a proximal portion of the distal tube 31 at a distal portion thereof, a main body section extending over a predetermined length toward the proximal side, and a proximal portion protruding beyond the shaft hub 30. In this embodiment, the shaft main body 33 has a structure wherein the distal portion fixed to the distal tube 31 is a small-diameter portion, whereas the main body section and the proximal portion are greater in outside diameter than the small-diameter portion. Besides, in this embodiment, the distal portion of the shaft body 33 is fixed to a side surface of the distal tube 31 by a heat-shrinkable tube 63.
The length of the shaft section 3 is preferably 400 to 2500 mm, particularly preferably 400 to 2200 mm. The outside diameter of the main body section of the shaft main body 33 is preferably 1.0 to 2.5 mm, particularly preferably 1.0 to 2.0 mm. The length of the distal tube 31 is preferably 10 to 400 mm, particularly preferably 50 to 350 mm, and the outside diameter of the distal tube 31 is preferably 0.2 to 2.0 mm, particularly preferably 0.4 to 1.7 mm. The inside diameter of the lumen 61 is preferably 0.1 to 1.8 mm, particularly preferably 0.3 to 1.0 mm.

The shaft main body 33 may be a solid body or a tubular body. Besides, the shaft main body 33 may be a coil shaft. The material forming the shaft bods 3 is preferably a material which, is hard and is flexible to a certain extent. Examples of the material which can be suitably used include stainless steel, superelastic metals, polyolefins such as polyethylene and polypropylene, nylon, polyethylene terephthalate, fluoro-polymer such as ETFE, PEEK (polyether ether ketone), and polyimides. Incidentally, the external surface of the shaft section 3 may be coated with a biocompatible material, particularly an antithrombogenic material. Examples of the antithrombogenic material which can be suitably used include polyhydroxyethyl methacrylate and hydorxyethyl methacrylate-styrene copolymers (e.g., HEMA-St-HEMA block copolymer).
Furthermore, the external surface of that part of the shaft section 3 which may protrude beyond the sheath 2 preferably lubricity. Therefore, the external surface of that part may be coated with a hydrophilic material such as poly(2-hydroxyethyl methacrylate), polyhydroxyethyl acrylate, hydroxypropyl cellulose, methyl vinyl ether-maleic anhydride copolymer, polyethylene glycol, polyacrylamide, polyvinylpyrrolidone, and dimethylacrylamide-glycidyl methacrylate copolymer, or may have such a hydrophilic material fixed thereto. In addition, the above-mentioned hydrophilic material may be applied or fixed to the whole part of the external surface of the shaft section 3. Further, the internal surface of the shaft section may also be coated with the above-mentioned hydrophilic material or may have the hydrophilic material fixed thereto, for enhancing slidability of the internal surface relative to a guide wire.

The shaft main body 33 passes through the sheath 2, to protrude beyond the proximal opening of the sheath 2. As shown in FIGS. 1, 2 and 9, the shaft hub 30 is firmly attached to a proximal portion of the shaft main body 33. In this embodiment, as shown in FIG. 8, a stationary ring 66 is fixed to the shaft main body 33. In addition, a proximal tube 34 extending further to the distal side relative to the shaft hub 30 is fixed to the shaft hub 30. Besides, a distal portion of the proximal tube 34 is fixed to the stationary ring 66. In addition, an elastic ring 69 is fixed to the proximal end (in the inside of the shaft hub 30) of the proximal tube 34. Further, in this embodiment, a second stationary ring 68 is provided at a position spaced a predetermined length to the distal side relative to the stationary ring 66. Besides, an intermediate tube 67 is disposed between the stationary ring 66 and the second stationary ring 68. The intermedate 67 is fixed to neither of the shaft main body 33 and the sheath tube 21, and can make contact with the stationary ring 66 the second stationary ring 68. With such an intermediate tube provided, favorable sliding of the sheath is ensured. The intermediate tube 67 is preferably a tube having a low-frictional surface. Specifically, the intermediate tube 67 is preferably a tube formed, for example, of polyolefins such as polyethylene and polypropylene, nylon, polyethylene terephthalate, or a fluoro-polymer such as PTFE and ETFE.

Besides, the distal contact section and the proximal contact section in the stent delivery system according to the present invention may be as shown in FIGS. 20 and 21. In the stent delivery system according to this embodiment, a tubular member 51 is fixed to the external surface of the inner tube body 31. The tubular member 51 is formed with, at a distal portion thereof, a distal protruding section 53 constituting the distal contact section, and is formed with, at a proximal portion thereof, a proximal protruding section 52 constituting the proximal contact section. The intermediate portion (between the distal protruding section 53 and the proximal protruding section 52) of the tubular member 51 is a small-diameter portion (annular recessed section) serving as a fixing section for fixation with the inner tube body 31. In other words, the distal portion and the proximal portion of the tubular member 51 are enlarged-diameter sections. The distal protruding section 53 is an annular protruding section, and has a predetermined width (axial length). The proximal protruding section 52 is an annular protruding section, and has a predetermined width (axial length). The use of such a tubular member 51 makes it easy to form the distal contact section and the proximal contact section. The outside diameters, axial lengths and the like of the distal protruding section 53 and the proximal protruding section 52 are the same as described above in relation to the distal protruding section 36 and the proximal protruding section 35. In addition, the tubular member 51 is preferably made from a radiopaque material. As the radiopaque material, the materials for forming the radiopaque marker described above can be suitably used.

Alternatively, the distal contact section and the proximal contact section in the stent delivery system according to the present invention may be as shown in FIG. 22. In the stent delivery system according to this embodiment, a coiled member 54 is fixed to the external surface of the inner tube body 31. The coiled member 54 is formed with a distal enlarged-diameter coil section 56 forming the distal contact section at a distal portion thereof, and is formed with a proximal enlarged-diameter coil section 55 forming the proximal contact section at a proximal portion thereof. The coiled member 54 is a coil formed from a single linear body, and its intermediate portion (between the distal enlarged-diameter coil section 56 and the proximal enlarged-diameter coil section 55) is a small-diameter portion serving as a fixing section for fixation with the inner tube body 31. In other words, the distal enlarged-diameter coil section 56 and the proximal enlarged-diameter coil section 55 of the coiled member 54 are enlarged-diameter sections. The distal enlarged-diameter coil section 56 is an annular protruding section so formed as not to make contact with the inner tube body 31, and has a predetermined axial length. The proximal enlarged-diameter coil section 55 is an annular protruding section so formed as not to make contact with the inner tube body 31, and has a predetermined axial length. The use of such a coiled member 54 makes it easy to form the distal contact section and the proximal contact section. The outside diameters, axial lengths and the like of the distal enlarged-diameter coil section 56 and the proximal enlarged-diameter coil section 55 are the same as described above in relation to the distal protruding section 36 and the proximal protruding section 35. In addition, the coiled member 54 is preferably made from a radiopaque material. As the radiopaque material, the materials for forming the radiopaque marker described above can be suitably used.

Alternatively, the distal contact section in the stent delivery system according to the present invention may be as shown in FIG. 23. In the stent delivery system according to this embodiment, a distal contact section 36a is a protruding section 36a formed of a tubular member having an opened-ring portion. The size of the opened-ring portion is preferably 1/6 to 1/2 of the whole circumference of the tubular member. Besides, the distal contact section may be a short semi-tubular member.
Further, the proximal contact section in the stent delivery system according to the present invention may be as shown in FIG. 24. In the stent delivery system according to this embodiment, a proximal contact section 35a is a protruding section 35a formed of a tubular member having an opened-ring portion. The size of the opened-ring portion is preferably 1/6 to 1/2 of the whole circumference of the tubular member. Besides, the proximal contact section may be a short semi-tubular member.
Furthermore, the distal contact section and the proximal contact section in the stent delivery system according to the present invention may be as shown in FIG. 25. In the stent delivery system according to this embodiment, a distal contact section 36a is a protruding section 36a formed of a tubular member having an opened-ring portion, and a proximal contact section 35a is also a protruding section 35a formed of a tubular member having an opened-ring portion. The size of the opened-ring portion is preferably 1/6 to 1/2 of the whole circumference of tubular member. Besides, the distal contact section and the proximal contact section may be a short semi-tubular member. Further, in the stent delivery system according to this embodiment, the opened-ruing portions of the tubular members or short semi-tubular members are preferably located at positions shifted from each other in the circumferential direction.

Besides, the distal contact section and/or the proximal contact section in the stent delivery system according to the present invention may be as shown in FIG. 26. In the stent delivery system according to this embodiment, a distal contact section 36b is a protruding section 36b formed of a tubular member having a plurality of opened-ring portions (in other words, a plurality of curved protrusions arranged in an annular pattern). Also, in the stent delivery system according to this embodiment, a proximal contact section 35b is a protruding section 35b formed of a tubular member having a plurality of opened-ring portions (in other words, a plurality of curved protrusions arranged in an annular pattern).
In all the embodiments described above, the tubular members having the opened-ring portions, the short semi-tubular members and the plurality of curved protrusions arranged in an annular pattern are each preferably made from a radiopaque material. As the radiopaque material, the materials for forming the radiopaque marker described above can be suitably used.

In addition, the proximal contact section in the stent delivery system according to the present invention may be as shown in FIG. 27. FIG. 27 is an illustration of the central portion of an inner tube body 3a (inclusive of a stent) in a stent delivery system according to another embodiment of the present invention. In this embodiment, the inner tube body 3a has a wire member extending from the proximal side toward the distal side, and the proximal contact section is formed of the distal end of the wire member.
The inner tube body 3a in this embodiment is substantially the same in configuration as the above-described inner tube body 3, except for the difference in the form of the distal portion of the shaft main body 33, as shown in FIG. 27.
As shown in FIG. 27, the inner tube body 3a includes a shaft main body 33 formed of a wire member, a distal tube 31 fixed on the distal side of the shaft main body 33, and a shaft hub (not shown) fixed to a proximal portion of the shaft main body 33. The stent 10 is so disposed as to envelope the distal tube 31. As shown in FIG. 27, the distal tube 31 is provided with a distal contact section (distal protruding section) 36 which is located within the proximal portion of the stent 10 and which does not make contact with the internal surface of a stent-containing tube body 2. The distal protruding section 36 is the same as the above-described one.
The shaft main body 33 formed of the wire member includes a main body section of which distal side is fixed to a proximal portion of the distal tube 31 and extending over a predetermined length to the proximal side, and a proximal portion protruding beyond the shaft hub. In this embodiment, the shaft main body 33 extends to the vicinity of the proximal end of the stent 10 beyond the part fixed to the distal tube 31, and a distalmost end 33a of the shaft main body 33 forms the proximal contact section for making contact with the proximal end of the stent 10. The distalmost end 33a of the shaft main body 33 is preferably a curved distal portion having a predetermined circumferential width. This promises assured contact with the proximal portion 16 of the stent. Furthermore, the distalmost end of the shaft main body 33 may be an annular section 33b, as in the inner tube 3b shown in FIG. 28. This promises more assured contact with the proximal portion 16 of the stent. The outside diameter of the annular section 33b is preferably the same as described above in relation to the proximal protruding section 35.

Besides, the proximal contact section in the stent delivery system according to the present invention may be as shown in FIG. 29. FIG. 29 is an illustration of the central portion of an inner tube body 3c (inclusive of a stent) in a stent delivery system according to a further embodiment of the present invention. In this embodiment, the inner tube body has an enlarged-diameter section, and the proximal contact section is formed of the enlarged-diameter section. Specifically, as shown in FIG. 29, the proximal contact section 33c is formed of the enlarged-diameter section of the inner tube 31 of the inner tube body 3c.
The inner tube body 3c in this embodiment is substantially the same in configuration as the above-described inner tube body 3, except for the difference in the form of the distal tube 31 at the vicinity of the proximal portion of the stent 10.
As shown in FIG. 29, the distal tube 31 has an enlarged-diameter section which is provided at a position a little on the proximal side relative to the proximal end of the stent 10 and which can make contact with the proximal portion 16 of the stent 10. In addition, in this embodiment, the enlarged-diameter section 33c of the distal tube 31 is an annular enlarged-diameter section, which retains its outside diameter over a predetermined length, before decreasing in diameter in a tapered manner. Incidentally, the distal tube 31 may extend to the proximal portion with the outside diameter of the enlarged-diameter section retained. Besides, the outside diameter of the enlarged-diameter section 33c is preferably the same as described above in relation to the proximal protruding section 35.

In addition, the proximal contact section in the stent delivery system according to the present invention may be as shown in FIG. 30. FIG. 30 is an illustration of a stent delivery system according to still another embodiment. In the stent delivery system 100 of this embodiment, an inner tube body has a tube member extending from the proximal side toward the distal side, and the proximal contact section is formed of the distal end of the tube member.
Specifically, as shown in FIG. 30, a shaft section (inner tube body) 3 includes a shaft main body 33, a distal tube 31 which is provided at the distal end of the shaft main body 33 and protrudes beyond the distal end of a sheath 2, a shaft hub 30 fixed to a proximal portion of the shaft main body) 33, and a tube body 38 which envelopes part of the shaft main body 33 and the distal tube 31 and extends to the vicinity of the proximal end of the stent 10.
As shown in FIG. 30, the tube body 38 has, at a position a little on the proximal side relative to the proximal end of the stent 10, a distal portion which can make contact with a proximal portion 16 of the stent 10. The distal portion of the tube body 38 constitutes the proximal contact section. The outside diameter of the distal portion of the tube body 38 is preferably the same as described above in relation to the proximal protruding section 35. Besides, the tube body 38 is fixed to the inner tube body at a proximal portion thereof. In this embodiment, as shown in FIG. 30, a stationary ring 66 is fixed to the shaft main body 33, and a proximal portion of the tube body 38 is fixed to the stationary ring 66. Incidentally, the tube body 38 may be fixed to the shaft hub (not shown). In addition, the tube body 38 is provided with a cutout for exposing a portion of the distal tube 31 near the proximal opening. The tube body 38 preferably has a low-frictional surface. Specifically, the tube body 38 is preferably a tube formed, for example, of a polyolefin such as polyethylene and polypropylene, nylon, polyethylene terephthalate, or a fluoro-polymer such as PTFE and ETFE.

Now, operation of the stent delivery system according to the present invention will be described below, referring to FIGS. 18 and 19.
The stent delivery system 1 with the guide wire 65 inserted and passed in the distal tube 31 is inserted into a blood vessel to be treated, to bring a stent to an indwelling site. In this state, the whole part of the stent 10 is in the state of being contained in the sheath 2. Then, the sheath 2 is slid toward the proximal side, whereby the stent 10 is exposed from the distal opening of the sheath 2, as shown in FIG. 18. In this instance, the proximal end of the stent 10 abuts on the proximal contact section 35 of the inner tube body 3 (distal tube 31), whereby the stent 10 is restricted in proximal movement, so that the stent 10 can be exposed. The stent 10 exposed from the sheath 2 expands by its self-expanding force to be restored into its pre-compression form. In the case where the disposing position of the stent 10 has to be readjusted, the sheath 2 is slid in the distal direction, whereby the stent is again contained into the sheath either partly or entirely, as shown in FIG. 19. In this instance, the proximal inwardly protruding section 17a of the stent 10 abuts on the distal contact section 36 of the inner tube body 3 (distal tube 31), whereby the stent 10 is restricted in distal movement, so that the stent 10 can be again contained into the sheath 2. Then, the stent-containing part is adjusted to an appropriate position, and thereafter, the sheath 2 is again slid toward the proximal side, whereby the stent 10 is exposed from the distal opening of the sheath 2. Subsequently, the sheath 2 is slid toward the proximal side until the proximal end of the stent is exposed, whereby the stent is completely discharged from the sheath, to be disengaged from the shaft section 3.

In addition, the stent to be used in the present invention may be as shown in FIG. 31. Like the above-described stent 10, this stent 70 is a so-called self-expandable stent which has a multiplicity of openings in the side surface thereof and which can be restored into its pre-compression shape by expanding outward when put indwelling in a living body. Further, the stent 70 has a distal portion and a proximal portion oriented respectively toward the distal side and the proximal side of a sheath 2. Furthermore, the stent 70 does not, except in its proximal portion, substantially have a bend free end oriented to the proximal side, and, after exposure of its distal portion from the sheath 2, its exposed distal portion can again be contained into the sheath 2 by moving the sheath 2 toward the distal side relative to a shaft section 3.

This stent 70 is a stent for indwelling in a living body that is formed in a substantially hollow cylindrical shape. The stent 70 includes wavy struts 73 and 74 which extend in the axial direction from one end to the other end of the stent 70 and which are arranged in plurality in the circumferential direction of the stent, and a plurality of connection struts 75 interconnecting each pair of wavy struts 73 and 74 adjacent to each other. The adjacent wavy struts 73 and 74 have pluralities of close sections and open sections. The connection struts 75 interconnect the close sections of the adjacent wavy struts 73 and 74, and are each provided with, at its central portion, a bent section 85 extending toward the distal side in the axial direction of the stent.

Particularly, in this stent 70, the pluralities of wavy struts 73 and 74 include a plurality of first wavy struts 73 having a plurality of upper points 73a and a plurality of lower points 73b, and a plurality of second wavy struts 74 having a plurality of upper points 74a and a plurality of lower points 74b and being each located between the first wavy struts. Each pair of the first wavy strut 73 and the second wavy strut 74 adjacent to each other are so disposed that the upper or lower points of the wavy strut on one side and the lower or upper points of the adjacent wavy strut on the other side substantially face each other, thereby forming the close sections. The connection struts 75 each interconnects the upper point 73a or lower point 73b of the first wavy strut 73 and the lower point 74b or upper point 74a of the second wavy strut which constitute a close section. In addition, the first wavy strut 73 and the second wavy strut 74 adjacent to each other are so disposed that the lower or upper points of the wavy strut on one side and the upper or lower points of the adjacent wavy strut on the other side are substantially opposite to each other, thereby forming the open sections.
This stent 70 is a so-called self-expandable stent which is formed in a substantially hollow cylindrical shape, is compressed toward a center axis thereof when inserted into a living body, and is restored into its pre-compression shape by expanding outward when put indwelling in the living body.

The first wavy struts 73 extend in an axial direction substantially parallel to the center axis of the stent. The first wavy struts 73 are arrayed in plurality in the circumferential direction of the stent. The number of the first wavy struts 73 is preferably not less than two, and particularly preferably three to eight. Furthermore, the plurality of first wavy struts 73 are preferably arranged at substantially regular angular intervals around the center axis of the stent.
In addition, in the stent 70 in this example, the first wavy strut 73 has a structure in which, except in both side portions, substantially the same waveform continues over a predetermined length. Specifically, the first wavy strut 73 has a structure in which, except in the vicinity of both end portions, substantially the same waveform, in other words, a wave of the same wavelength and the same amplitude, continues. In the case where the first wavy struts 73 have the same waveform throughout substantially the whole part thereof, the wavelength, though different depending on the outside diameter of the stent, is preferably, 0.5 to 8.0 mm, particularly preferably 2.0 to 4.0 mm, and the amplitude is preferably 0.1 to 10.0 mm, particularly preferably 0.3 to 3.0 mm.

The second wavy struts 74 extend in an axial direction substantially parallel to the center axis of the stent. The second wavy struts 74 are arrayed in plurality in the circumferential direction of the stent, and each of the second wavy struts 74 is arranged between the first wavy struts. The number of the second wavy struts is preferably not less than two, and particularly preferably three to eight. Furthermore, the plurality of second wavy struts 74 are preferably arranged at substantially regular angular intervals around the center axis of the stent. In addition, the number of the second wavy struts 74 is the same as the number of the first wavy struts.
Besides, in this stent 70, the second wavy strut 74 has a structure in which, except in both side portions, substantially the same waveform continues over a predetermined length. Specifically, the second wavy strut 74 has a structure in which, except in the vicinity of both end portions, substantially the same waveform, in other words, a wave of the same wavelength and the same amplitude, continues. In the case where the second wavy struts 74 have the same waveform throughout substantially the whole part thereof, the wavelength, though different depending on the outside diameter of the stent, is preferably 0.5 to 8.0 mm, particularly preferably 2.0 to 4.0 mm, and the amplitude is preferably 0.1 to 10.0 mm, particularly preferably 0.3 to 3.0 mm.

Furthermore, in this stent 70, the first wavy struts 73 and the second wavy struts 74 have substantially the same waveform. Specifically, in the stent 70, the first wavy strut 73 and the second wavy strut 74 have substantially the same wavelength and substantially the same amplitude; in addition, the second wavy struts 74 are shifted by about one half the wavelength in the axial direction of the stent, in relation to the first wavy struts 73.
Therefore, as shown in FIG. 31, the first wavy strut 73 and the second wavy strut 74 adjacent to each other are so disposed that the upper points 73a or lower points 73b of the first wavy strut 73 and the lower points 74b or upper points 74a of the second wavy struts 74 substantially face each other (or are substantially opposed to each other), thereby forming the close sections and the open sections. In other words, in this stent 70, the first wavy strut 73 and the second wavy strut 74 adjacent to each other are so disposed that their upper points do not face each other (are not opposed to each other) and their lower points do not face each other (are not opposed to each other), and, accordingly, the close sections and the open sections are provided alternately along the axial direction.
In addition, in the stent in this example, the wavy struts 73 and 74 are all the same in length, except for both end sections. Therefore, when the stent is compressed in the radial direction, each of the struts approaches parallelism with the axial direction, and, since the struts are the same in length, the stent is reduced in diameter favorably, without the struts stiffening in the axial direction. Besides, in the stent in this example, the wavy struts 73 and 74 are, except at both ends thereof, arranged at regular angular intervals around the center axis of the stent. When the stent is compressed in the radial direction, therefore, the gaps between the struts are equally reduced, so that the stent is favorably contracted without mutual overlapping of the struts.

As shown in FIG. 31, the stent 70 has the connection struts 75 which interconnect the close sections of the adjacent wavy struts 73 and 74 and each of which is provided at a central portion thereof with the bent section 85 extending toward the distal side in the axial direction of the stent. The axial length of the connection strut 75, though different depending on the outside diameter of the stent, is preferably 0.1 to 3.0 mm, particularly preferably 0.5 to 2.0 mm. In addition, the connection struts 75 are symmetric with respect to the center axis of the stent 70 and with respect to the vertex of the bent section 85. Besides, in this stent 70, substantially all of the plurality of proximate sections of the first wavy strut 73 and the second wavy strut 74 adjacent to each other are interconnected by the connection struts 75, respectively. The bent sections 85 of the connection struts 75 are each located in the vicinity of the open sections formed between the wavy struts 73 and 74. In addition, the bent section 85 of the connection strut 75 is a free end extending in the distal direction of the stent 70. In the stent 70 in this example, the connection struts 75 are provided in plurality and in series along the axial direction of the stent. Besides, the connection struts 75 are provided in plurality in the circumferential direction of the stent.
The stent 70 in this example is provided with, at the distal portion thereof, bent sections 72 each formed by coupling distal portions of the first wavy strut 73 and the second wavy strut 74, and bent sections 76 each formed by coupling the distal portion of a linear section 63 connected to the first wavy strut 73 through a branching section 61 and the distal portion of a linear section 64 connected to the second wavy strut 74 through a branching section 62. The bent sections 72 and the bent sections 76 are provided alternately along the circumferential direction of the stent 70. In addition, a radiopaque marker 77 is mounted to each of the bent sections 76. Besides, the bent sections 76 provided with the radiopaque markers 77 are located on the stent distal side relative to the bent sections 72,

In addition, the stent 70 is provided at the proximal portion thereof with bent sections 79 each formed by coupling proximal portions of the first wavy strut 73 and the second wavy strut 74, and with bent sections 78 each formed by coupling the proximal portion of a linear section 83 connected to the first wavy strut 73 through a branching section 81 and the proximal portion of a linear section 84 connected to the second wavy strut 74 through a branching section 82. The bent sections 79 and the bent sections 78 are provided alternately along the circumferential direction of the stent 70. In other words, in the stent 70, the bent sections 78 and the bent sections 79 form the proximal portion oriented to the proximal side of the sheath (stent-containing member). A radiopaque marker 77 is mounted to each of the bent sections 78. Incidentally, in this stent 70, the radiopaque markers 77 form a proximal inwardly protruding section 77a which will be described later. Besides, the bent sections 78 provided with the radiopaque markers 77 are located on the stent proximal side relative to the bent sections 79. In addition, this stent 70 does not have a free end oriented to the proximal side of the stent, except for the bent sections 78 and 79. Therefore, at the time of moving the sheath toward the distal side relative to the inner tube body after a distal portion of the stent is partly exposed from the sheath, the absence of any free end (of the stent) extending toward the sheath (stent-containing member) ensures that the stent can be again contained into the sheath (stent-containing member) without being caught on the sheath. Besides, in this stent 70, the linear sections 83 and 84 constituting the bent sections 78 at the proximal portion are axially longer than the linear sections 63 and 64 constituting the bent sections 76 at the distal portion. The stent 70 is inserted into a living body from the distal side (the bent section 76 side) thereof and is put indwelling in the living body.

In addition, the radiopaque marker 77 envelopes substantially the whole part or a part of the two frame portions constituting the bent section. The radiopaque marker 77 is a thin rectangular parallelepiped body, contains therein the two frame portions, and is depressed in the central portion thereof, to be thereby fixed to the two frame portions. As the material forming the radiopaque marker, there can be suitably used either one (simple substance) or at least two (alloy) selected from the group consisting of iridium, platinum, gold, rhenium, tungsten, palladium, rhodium, tantalum, silver, ruthenium, and hafnium. The length of the marker is preferably 0.1 to 4.0 mm, particularly preferably 0.3 to 1.0 mm. The material thickness of the marker is preferably 0.01 to 0.30 mm, particularly preferably 0.03 to 0.10 mm.

FIG. 32 is an illustration of a stent delivery system using the stent for indwelling in a living body of FIG. 31.
As shown in FIG. 32, the stent 70 has proximal inwardly protruding sections 77a constituted by the radiopaque markers 77. The stent 70 is so disposed that only the proximal inwardly protruding sections 77a, in other words, only the bent sections 78, are located between the distal contact section 36 and the proximal contact section 35 of the inner tube body 3. In addition, the stent 70 is so disposed that the bent sections 79, each provided between the adjacent bent sections 78, are located on the distal side relative to the distal contact section 36. This configuration ensures that when the stent 70 is compressed in the radial direction, the adjacent radiopaque markers 77 are prevented from making contact or overlapping with each other, so that the stent 70 is contracted favorably. Besides, the proximal inwardly protruding sections 77a of the stent can be easily disposed between the distal contact section 36 and the proximal contact section 35.

### Industrial Applicability

The stent delivery system according to the present invention resides is as follows.
(1) A stent delivery system including: a stent having a multiplicity of side wall openings, formed in a substantially hollow cylindrical shape, compressed toward a center axis thereof when inserted into a living body, and capable of being restored into a pre-compression shape by expanding outward when put indwelling in the living body; an inner tube body having a guide wire lumen; and a stent-containing tube body which contains the stent in a distal portion thereof, the stent being so disposed as to cover a distal portion of the inner tube body, and the stent being dischargeable by moving the stent-containing tube body toward a proximal side relative to the inner tube body,
   wherein the inner tube body includes a distal contact section which is located in a proximal portion of the stent and which does not enter the side wall openings of the stent, and a proximal contact section which is disposed at a position rear of a proximal end of the stent and close to the distal contact section and which is capable of making contact with the proximal end of the stent; the stent is provided with a proximal inwardly protruding section capable of making contact with the distal contact section of the inner tube body; the stent is so disposed that the proximal inwardly protruding section is located between the distal contact section and the proximal contact section of the inner tube body; and the stent is provided with a distal portion and the proximal portion oriented respectively to a distal side and the proximal side of the stent-containing tube body and does not, except in the proximal portion thereof, have a bend free end which protrudes to the proximal side.

Besides, embodiments of the present invention may be as follows.
(2) The stent delivery system according to the above paragraph (1), wherein the distal contact section does not substantially make contact with an internal surface of the stent.
(3) The stent delivery system according to the above paragraph (1) or (2), wherein the distal contact section is formed of a protruding section provided at an external surface of the inner tube body.
(4) The stent delivery system according to any of the above paragraphs (1) to (3), wherein the proximal contact section is formed of a protruding section provided at an external surface of the inner tube body.
(5) The stent delivery system according to the above paragraph (4), wherein the proximal contact section has a protrusion height greater than a protrusion height of the distal contact section.
(6) The stent delivery system according to any of the above paragraphs (1) to (5), wherein the proximal contact section does not make contact with an internal surface of the stent-containing tube body.
(7) The stent delivery system according to any of the above paragraphs (1) to (6), wherein the distal contact section and/or the proximal contact section is formed of a tubular member fixed to an external surface of the inner tube body.
(8) The stent delivery system according to any of the above paragraphs (1) to (6), wherein the distal contact section and the proximal contact section are formed of a tubular member fixed to an external surface of the inner tube body, and the tubular member has a distal protruding section forming the distal contact section and a proximal protruding section forming the proximal contact section.
(9) The stent delivery system according to any of the above paragraphs (1) to (6), wherein the distal contact section and the proximal contact section are formed of a coiled member fixed to an external surface of the inner tube body, and the coiled member has a distal enlarged-diameter coil section forming the distal contact section and a proximal enlarged-diameter coil section forming the proximal contact section.
(10) The stent delivery system according to any of the above paragraphs (1) to (6), wherein the distal contact section and/or the proximal contact section is formed of one or a plurality of protrusions.
(11) The stent delivery system according to any of the above paragraphs (1) to (6), wherein the distal contact section and/or the proximal contact section is formed of a tubular member or short semi-tubular member which has an opened-ring portion and which is fixed to an external surface of the inner tube body.
(12) The stent delivery system according to any of the above paragraphs (1) to (11), wherein the distal contact section and the proximal contact section are different from each other in axial length.

(13) The stent delivery system according to the above paragraph (1) or (2), wherein the inner tube body has a wire member or tube member which extends from the proximal side to the distal side, and the proximal contact section is formed of a distal portion of the wire member or tube member.
(14) The stent delivery system according to the above paragraph (1) or (2), wherein the inner tube body has an enlarged-diameter section, and the proximal contact section is formed of the enlarged diameter section.
(15) The stent delivery system according to any of the above paragraphs (1) to (14), wherein the distal contact section and the proximal contact section are made from a radiopaque material.
(16) The stent delivery system according to any of the above paragraphs (1) to (15), wherein the proximal inwardly protruding section is formed of a radiopaque marker mounted to the proximal portion of the stent.
(17) The stent delivery system according to any of the above paragraphs (1) to (16), wherein the proximal inwardly protruding section is formed of a sheet-formed member or wire-formed member which is wound around the proximal portion of the stent, and the sheet-formed member or wire-formed member has an inner overlap section protruding to an inner side of the stent.
(18) The stent delivery system according to any of the above paragraphs (1) to (15), wherein the proximal inwardly protruding section is constituted by a thick-walled section formed at the proximal portion of the stent.
(19) The stent delivery system according to any of the above paragraphs (1) to (18), wherein the stent is provided at the distal portion thereof with a distal inwardly protruding section.
(20) The stent delivery system according to the above paragraph (19), wherein the distal inwardly protruding section is formed of a radiopaque marker mounted to the distal portion of the stent.
(21) The stent delivery system according to any of the above paragraphs (1) to (20), wherein after a distal portion of the stent is exposed from the stent-containing tube body, the exposed portion of the stent can be again contained into the stent-containing tube body by moving the stent-containing tube body toward the distal side relative to the inner tube.
(22) The stent delivery system according to any of the above paragraphs (1) to (21), wherein the stent includes wavy struts which each extend in an axial direction from one end to the other end of the stent and which are arrayed in plurality in a circumferential direction of the stent, and a plurality of connection struts by which adjacent ones of the wavy struts are interconnected; and an end portion of each wavy strut is connected to an end portion of one of the adjacent wavy struts.
(23) The stent delivery system according to any of the above paragraphs (1) to (22), wherein the inner tube body includes a distal tube having the guide wire lumen, and an inner tube main body having a distal portion fixed to the proximal side of the distal tube.

## Claims

1. A stent delivery system comprising: a stent having a multiplicity of side wall openings, formed in a substantially hollow cylindrical shape, compressed toward a center axis thereof when inserted into a living body, and capable of being restored into a pre-compression shape by expanding outward when put indwelling in the living body; an inner tube body having a guide wire lumen; and a stent-containing tube body which contains the stent in a distal portion thereof, the stent being so disposed as to cover a distal portion of the inner tube body, and the stent being dischargeable by moving the stent-containing tube body toward a proximal side relative to the inner tube body,
wherein the inner tube body includes a distal contact section which is located in a proximal portion of the stent and which does not enter the side wall openings of the stent, and a proximal contact section which is disposed at a position rear of a proximal end of the stent and close to the distal contact section and which is capable of making contact with the proximal end of the stent; the stent is provided with a proximal inwardly protruding section capable of making contact with the distal contact section of the inner tube body; the stent is so disposed that the proximal inwardly protruding section is located between the distal contact section and the proximal contact section of the inner tube body; and the stent is provided with a distal portion and the proximal portion oriented respectively to a distal side and the proximal side of the stent-containing tube body and does not, except in the proximal portion thereof, have a bend free end which protrudes to the proximal side.

2. The stent delivery system according to claim 1, wherein the distal contact section does not substantially make contact with an internal surface of the stent.

3. The stent delivery system according to claim 1 or 2, wherein the distal contact section is formed of a protruding section provided at an external surface of the inner tube body.

4. The stent delivery system according to any of claims 1 to 3, wherein the proximal contact section is formed of a protruding section provided at an external surface of the inner tube body.

5. The stent delivery system according to claim 4, wherein the proximal contact section has a protrusion height greater than a protrusion height of the distal contact section.

6. The stent delivery system according to any of claims 1 to 5, wherein the proximal contact section does not make contact with an internal surface of the stent-containing tube body.

7. The stent delivery system according to any of claims 1 to 6, wherein the distal contact section and/or the proximal contact section is formed of a tubular member fixed to an external surface of the inner tube body.

8. The stent delivery system according to any of claims 1 to 6, wherein the distal contact section and the proximal contact section are formed of a tubular member fixed to an external surface of the inner tube body, and the tubular member has a distal protruding section forming the distal contact section and a proximal protruding section forming the proximal contact section.

9. The stent delivery system according to any of claims 1 to 6, wherein the distal contact section and the proximal contact section are formed of a coiled member fixed to an external surface of the inner tube body, and the coiled member has a distal enlarged-diameter coil section forming the distal contact section and a proximal enlarged-diameter coil section forming the proximal contact section.

10. The stent delivery system according to any of claims 1 to 6, wherein the distal contact section and/or the proximal contact section is formed of one or a plurality of protrusions.

11. The stent delivery system according to any of claims 1 to 6, wherein the distal contact section and/or the proximal contact section is formed of a tubular member or short semi-tubular member which has an opened-ring portion and which is fixed to an external surface of the inner tube body.

12. The stent delivery system according to any of claims 1 to 11, wherein the distal contact section and the proximal contact section are different from each other in axial length.

13. The stent delivery system according to claim 1 or 2, wherein the inner tube body has a wire member or tube member which extends from the proximal side to the distal side, and the proximal contact section is formed of a distal portion of the wire member or tube member.

14. The stent delivery system according to claim 1 or 2, wherein the inner tube body has an enlarged-diameter section, and the proximal contact section is formed of the enlarged diameter section.

15. The stent delivery system according to any of claims 1 to 14, wherein the distal contact section and the proximal contact section are made from a radiopaque material.

16. The stent delivery system according to any of claims 1 to 15, wherein the proximal inwardly protruding section is formed of a radiopaque marker mounted to the proximal portion of the stent.

17. The stent delivery system according to any of claims 1 to 16, wherein the proximal inwardly protruding section is formed of a sheet-formed member or wire-formed member which is wound around the proximal portion of the stent, and the sheet-formed member or wire-formed member has an inner overlap section protruding to an inner side of the stent.

18. The stent delivery system according to any of claims 1 to 15, wherein the proximal inwardly protruding section is constituted by a thick-walled section formed at the proximal portion of the stent.

19. The stent delivery system according to any of claims 1 to 18, wherein the stent is provided at the distal portion thereof with a distal inwardly protruding section.

20. The stent delivery system according to claim 19, wherein the distal inwardly protruding section is formed of a radiopaque marker mounted to the distal portion of the stent.

21. The stent delivery system according to any of claims 1 to 20, wherein after a distal portion of the stent is exposed from the stent-containing tube body, the exposed portion of the stent can be again contained into the stent-containing tube body by moving the stent-containing tube body toward the distal side relative to the inner tube.

22. The stent delivery system according to any of claims 1 to 21, wherein the stent comprises wavy struts which each extend in an axial direction from one end to the other end of the stent and which are arrayed in plurality in a circumferential direction of the stent, and a plurality of connection struts by which adjacent ones of the wavy struts are interconnected; and an end portion of each wavy strut is connected to an end portion of one of the adjacent wavy struts.

23. The stent delivery system according to any of claims 1 to 22, wherein the inner tube body comprises a distal tube having the guide wire lumen, and an inner tube main body having a distal portion fixed to the proximal side of the distal tube.
